# EUROPÄISCHE PATENTSCHRIFT

(11) **EP 3 010 488 B1**
(45) Veröffentlichungstag und Bekanntmachung des Hinweises auf die Patenterteilung: **05.05.2021**
(21) Anmeldenummer: 14730932.2
(22) Anmeldetag: 18.06.2014
(51) Int. Cl.: A61K 9/127, A61K 31/704, C07F 9/09, A61P 35/00, A61P 9/10, A61P 7/02, A61P 29/00

(54) **STEREOSPEZIFISCHE LIPIDE ZUR LOKOREGIONÄREN THERAPIE MIT LANGZEITZIRKULIERENDEN STIMULI-SENSITIVEN NANOCARRIERSYSTEMEN**
STEREOSPECIFIC LIPIDS FOR LOCOREGIONAL THERAPY WITH LONG-TERM CIRCULATING STIMULI-SENSITIVE NANOCARRIER SYSTEMS
LIPIDES STÉRÉOSPÉCIFIQUES POUR LA THÉRAPIE LOCORÉGIONALE COMPRENANT DES SYSTÈMES NANOPORTEURS SENSIBLES AUX STIMULI CIRCULANT LONGTEMPS

(30) Priorität: 18.06.2013 EP 13172469
(43) Veröffentlichungstag der Anmeldung: 27.04.2016
(73) Patentinhaber: Thermosome Gmbh, 82152 Planegg/Martinsried (DE)
(72) Erfinder: EIBL, Hansjörg, 37120 Bovenden (DE)
(74) Vertreter: Dey, Michael
(86) Internationale Anmeldenummer: PCT/EP2014/062849
(87) Internationale Veröffentlichungsnummer: WO 2014/202680

(56) Entgegenhaltungen:
- EP-A1- 0 319 136
- WO-A1-97/30058
- WO-A1-2004/026282
- WO-A2-02/064116
- HOSSANN ET AL: "In vitro stability and content release properties of phosphatidylglyceroglycerol containing thermosensitive liposomes", BIOCHIMICA ET BIOPHYSICA ACTA (BBA) - BIOMEMBRANES, ELSEVIER, AMSTERDAM, NL, Bd. 1768, Nr. 10, 9. Oktober 2007 (2007-10-09), Seiten 2491-2499, XP022293149, ISSN: 0005-2736, DOI: 10.1016/J.BBAMEM.2007.05.021 in der Anmeldung erwähnt
- A. S. L. DERYCKE ET AL: "Transferrin-Conjugated Liposome Targeting of Photosensitizer AlPcS4 to Rat Bladder Carcinoma Cells", JNCI JOURNAL OF THE NATIONAL CANCER INSTITUTE, Bd. 96, Nr. 21, 2. November 2004 (2004-11-02), Seiten 1620-1630, XP055140924, ISSN: 0027-8874, DOI: 10.1093/jnci/djh314
- LANDON C D ET AL: "Nanoscale drug delivery and hyperthermia: The materials design and preclinical and clinical testing of low temperature-sensitive liposomes used in combination with mild hyperthermia in the treatment of local cancer", OPEN NANOMEDICINE JOURNAL 2011 BENTHAM SCIENCE PUBLISHERS B.V. NLD, Bd. 3, Nr. SPEC. ISSUE, 25. Mai 2011 (2011-05-25), Seiten 38-64, XP55140933, ISSN: 1875-9335

## Beschreibung

Die vorliegende Anmeldung betrifft stereospezifische Lipide zur lokoregionären Therapie mit langzeitzirkulierenden stimuli-sensitiven Nanocarriersystemen.

Die vorliegende Anmeldung betrifft insbesondere ein thermosensitives Liposom zur Behandlung von Tumoren, insbesondere von Harnblasentumoren und anderen lokalisierten Tumoren.

Die vorliegende Anmeldung betrifft insbesondere die Behandlung des Harnblasentumors mit Langzeit zirkulierenden thermosensitiven Liposomen. Der Therapieansatz ist aber von genereller klinischer Bedeutung, denn er kann allgemein zur Therapie von Erkrankungen beim Menschen und beim Tier durch lokoregionäre Freisetzung von Arzneistoffen und insbesondere auch bei anderen Tumorerkrankungen angewandt werden, die durch die lokalisierte Ansiedlung von Tumorzellen in Organen und Körpergewebe verursacht werden.

Die Anmeldung betrifft weiterhin Phospholipide mit einheitlicher und natürlicher Konfiguration, die insbesondere als Bestandteile der erfindungsgemäßen Nanocarriersysteme, z.B. als Bestandteile von Liposomen, eingesetzt werden können.

WO 2004/026282 A1 beschreibt ein thermolabiles Liposom mit geregelter Freigabetemperatur. Die beschriebenen Liposomen sind im Wesentlichen aus mindestens einem Phosphatidylcholin mit einer Hauptumwandlungstemperatur im Bereich von 0 bis 80°C und mehr als 15 bis 70 Gew.% Phosphatidyloligogylcerin gebildet.

WO 02/064116 A2 beschreibt ein thermolabiles Liposom mit geregelter Freigabetemperatur für den Liposomeninhalt, welches im Wesentlichen aus mindestens einem Phosphatidylcholin mit einer Hauptumwandlungstemperatur, die im Bereich von 0 bis 80°C liegt und 2 bis 15 Gew.% Phosphatidyloligoglycerin gebildet ist.

M. Hossann et al., Biochimica et Biophysica Acta 1768 (2007) 2491-2499 beschreibt Untersuchungen zur in vitro temperaturabhängigen Freisetzung hydrophiler Substanzen aus Phosphatidyldiglycerin-enthaltenden thermosensitiven Lipsomen.

A. S. L. Derycke et al., J. Nat. Canc. Inst. Vol. 96, No. 21, Nov. 3, 2004, 1620-1630 beschreibt Transferrin-konjugiertes Liposomtargeting des Photosensitizers AlPcS₄ zu Rattenblasenkarzinomzellen.

C. D. Landon et al., The Open Nanomedicine Journal, 2011, 3, 38-64 beschreibt Formulierungsentwicklung, in vitro Charakterisierung, sowie präklinische und klinische Untersuchungen von Niedertemperatur-sensitiven Liposomen basierend auf Lysolipiden und PEGylierten Lipiden, eingesetzt in Kombination mit Hyperthermie bei der Behandlung von lokalen Tumoren.

WO 97/30058 beschreibt Phosphatidyloligoglycerine und deren Einsatz zur Bildung von Cholesterin-haltigen Liposomen mit verlängerter Halbwertszeit in Blut.

EP 0 319 136 A1 beschreibt Phosphatidylglycerin-enthaltende Liposomen.

Das Harnblasenkarzinom ist eine häufige onkologische Erkrankung. Weltweit versterben jährlich ca. 130 000 Menschen an einem Harnblasenkarzinom. Männer erkranken doppelt so häufig wie Frauen, wobei das mittlere Erkrankungsalter bei 69 Jahren für Männer und bei 73 Jahren bei Frauen liegt. Etwa 95 % der Blasenkarzinome sind urothelialen Ursprungs. Klinisch ist vor allem die Unterscheidung zwischen *low-grade* und einem *high-grade* Tumoren von Bedeutung, da für letztere ein sehr hohes Rezidiv- und Progressionsrisiko besteht. Der histologische Nachweis eines Blasentumors mit Bestimmung der Infiltrationstiefe erfolgt durch eine transurethrale Resektion des Tumors (TURB). Bei allen *high-grade* und T1-Tumoren (d.h. mit Infiltration der Lamina propria) ist die transurethrale Nachresektion zwei bis sechs Wochen nach dem initialem Eingriff nötig. Bei Invasion des Tumors in die Muskelschicht besteht die Indikation zur radikalen Zystektomie.

Zusätzlich zum chirurgischen Vorgehen ist die intravesikale Chemotherapie, d.h. die Instillation mit Mitomycin C, Doxorubicin oder Epirubicin ein bewährtes Verfahren zur Therapie von Harnblasentumoren. Dabei wird nach der Resektion eines *low-risk* Tumors lediglich die Frühinstillation (innerhalb der ersten 6 Stunden postoperativ) empfohlen, wohingegen bei *intermediate risk* und *high risk* zusätzlich eine Erhaltungstherapie mit mehrmaligen Chemotherapieinstillationen empfohlen wird. Die Instiallationen erfolgen z.T. in wöchentlichen Abständen und können über mehrere Monate fortgeführt werden. Eine Alternative zur Instillation von Chemotherapie stellt die Instillation von BCG (Bacile Calmette Guerin) dar.

Trotz TURB und intravesikaler Chemotherapie besteht ein hohes Rezidivrisiko für oberflächliche Harnblasenkarzinome. Limitierend für die Wirksamkeit der intravesikalen Chemotherapie ist die eingeschränkte Tiefenpenetration in die Blase, da die Urothelschicht eine sehr effektive Barriere für Zytostatika darstellt. Von der Innenseite der Blase aus betrachtet ist die Blasenwand durch folgende Gewebe aufgebaut: Urothel, Lamina propria, Tunica muscularis und Adventitia. Nach Instillation von Zytostatika in die Blase nimmt die Gewebekonzentration mit zunehmender Gewebetiefe semilogarithmisch ab. Im Menschen konnte gezeigt werden, dass ab einer Infiltrationstiefe von 500 µm nur noch 50 % der initialen Gewebekonzentration vorliegen (Wientjes MG et al. Penetration of mitomycin C in human bladder. Cancer Res. 1993). An einem Hundemodell konnte gezeigt werden, dass zytotoxische Konzentrationen für Mitomycin C lediglich innerhalb der Urothelschicht erreicht werden können, wohingegen nur in 20 % der Fälle ausreichend hohe Konzentrationen in der Lamina propria und weniger als 20 % der Fälle in der Tunica muscularis erreicht wurden. So war in dieser Untersuchung in einer Gewebetiefe von 2000 µm eine mediane Konzentration von Mitomycin C 1 µg/g (n=24) nachweisbar. In einer Gewebetiefe von 2000 - 3000 µm waren bei 18/24 Hunden kein Mitomycin C mehr nachweisbar (Wientjes MG et al. Bladder wall penetration of intravesical mitomycin C in dogs. Cancer Res. 1991). Zusammenfassend ist die Wirksamkeit der intravesikalen Chemotherapie sehr stark eingeschränkt durch eine unzureichende Tiefenpenetration der eingesetzten Zytostatika.

Eine Aufgabe der Erfindung war es deshalb, eine Behandlungsmöglichkeit von lokalisierten Erkrankungen und bevorzugt von Tumoren und insbesondere von Harnblasentumoren und anderen lokalisierten Tumoren zur Verfügung zu stellen.

Diese Aufgabe wird erfindungsgemäß gelöst durch ein stimuli-sensitives Nanocarriersystem zur Verwendung zur lokoregionären Therapie, welches mindestens ein Phosphatidyloligoglycerin der Formel (IIa) 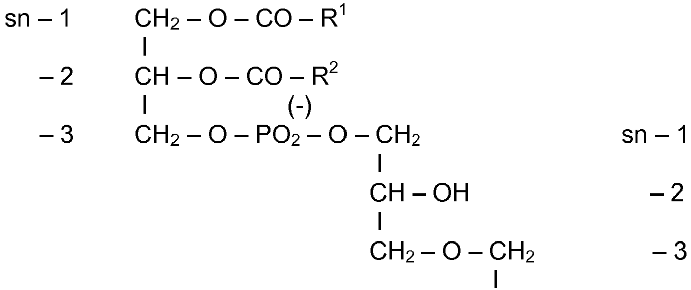 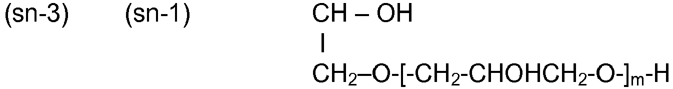 umfasst, worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.

Bei dem erfindungsgemäßen Nanocarriersystem handelt es sich vorzugsweise um Liposomen. Es können aber auch andere Nanocarriersysteme eingesetzt werden. Erfindungswesentlich ist, dass die Nanocarriersysteme, insbesondere Liposomen, stimuli-sensitiv sind. Die erfindungsgemäßen stimuli-sensitiven Nanocarriersysteme, insbesondere stimuli-sensitiven Liposomen, geben einen eingeschlossenen oder assoziierten Wirkstoff in Reaktion auf die Ausübung eines Stimulus frei. Geeignete Stimuli oder Energiequellen sind vorzugsweise Wärme, Radiofrequenz, z.B. radiative Oberflächen- und Tiefenhyperthermiesysteme oder Blasenhyperthermiesysteme, Ultraschall, wie z.B. hochfokussierter Ultraschall (HIFU) oder niedrigintensiver Ultraschall (LIFU), Licht, Laser, Konduktion durch erwärmte Flüssigkeit oder andere physikalische Prinzipien, die zu einer lokoregionären Erwärmung führen und/oder Membranen, insbesondere Membranen umfassend Phospholipide, destabilisieren.

Mit einer lokoregionären Therapie, insbesondere durch die gezielte und schnelle Freisetzung von in den stimuli-sensitiven Nanocarriersystemen eingeschlossenen Wirkstoffen, können lokalisierte Erkrankungen zielgerichtet behandelt werden ohne Schädigung von gesundem Gewebe. Zudem wird durch die gezielte Freisetzung von Wirkstoffen die systemische Belastung minimal gehalten.

Die Erfindung betrifft insbesondere ein stimuli-sensitives, bevorzugt ein thermosensitives Liposom zur Verwendung zur Behandlung von Tumoren und insbesondere von Harnblasentumoren. Durch Einsatz von thermosensitiven Liposomen kann ein in den Liposomen eingeschlossener Wirkstoff durch gezielte Erwärmung der Liposomen freigesetzt werden. Deshalb eignen sich solche thermosensitiven Liposomen besonders gut zur lokalen Therapie von Tumoren und insbesondere von Harnblasentumoren bzw. Harnblasenkarzinomen. Die Freisetzung des in den Liposomen eingeschlossenen Wirkstoffs kann gezielt an der gewünschten Stelle im Körper, z.B. in der Harnblasenwand, induziert werden, wodurch der Wirkstoff, insbesondere ein Zytostatikum, unmittelbar an der gewünschten Stelle freigesetzt werden kann.

Besonders bevorzugt umfasst das erfindungsgemäß eingesetzte stimuli-sensitive Nanocarriersystem, insbesondere ein thermosensitives Liposom, (i) mindestens ein Phosphatidylcholin mit einer Hauptumwandlungstemperatur von 0 °C bis 80 °C und (ii) mindestens ein Phosphatidylol igoglycerin.

Durch den Einsatz von Phosphatidyloligoglycerinen ist es gelungen thermosensitive Lipsomen mit verlängerter Blutzirkulationszeit herzustellen (WO 2002/064116 und WO 2004/026282). Diese Liposomen sind z.B. unter physiologischen Bedingungen (37 - 38 °C) im Blutkreislauf sehr stabil und setzen einen zuvor in das Liposom eingeschlossenen Wirkstoff nicht bzw. nur unwesentlich frei (Lindner LH et al. Novel temperature-sensitive liposomes with prolonged circulation time. Clin Cancer Res. 2004; Hossann M et al. In vitro stability and content release properties of phosphatidylglyceroglycerol containing thermosensitive liposomes. Biochim Biophys Acta. 2007). Über einen Zeitraum von 2 Stunden verbleibt der Großteil der Liposomen in der Blutzirkulation und steht somit einer wärmegesteuerten Wirkstofffreisetzung zur Verfügung. Bedingt durch die rasche Freisetzungskinetik dieser Liposomen mit einer wärmeinduzierten Wirkstofffreisetzung innerhalb von wenigen Sekunden, vorzugsweise < 15 s, mehr bevorzugt < 10 s kann ein zuvor eingeschlossener Wirkstoff durch eine gezielte Erwärmung der Liposomen auf Temperaturen > 39 °C, vorzugsweise 40 - 42 °C, schlagartig freigesetzt werden.

Durch die hohe Stabilität der erfindungsgemäßen Liposomen in der systemischen Zirkulation mit nur sehr geringer unspezifischer Wirkstofffreisetzung eignen sich diese Liposomen in ganz besonderer Weise für die lokale Therapie von Tumoren, insbesondere von lokalisierten Tumoren und besonders bevorzugt des Harnblasenkarzinoms. So kann nach intravenöser Applikation der erfindungsgemäßen Liposomen durch die gezielte Erwärmung der gewünschten Stelle, z.B. der Harnblasenwand, eine Freisetzung eines zuvor eingeschlossenen Wirkstoffs an der gewünschten Stelle, z.B. in der Harnblasenwand, induziert werden.

Durch dieses Verfahren kann die natürliche Barriere des Urothels umgangen werden und es können erstmals hohe Gewebespiegel des eingeschlossenen Medikamentes bis hin zur Tunica muscularis erreicht werden.

Erfindungsgemäß werden Tumore, insbesondere solide oder/und lokalisierte Tumore behandelt. Mit den erfindungsgemäßen Liposomen können insbesondere oberflächlich gelegene Tumore und Metastasen behandelt werden, da diese auf einfache Weise erwärmt werden können. Es können aber auch andere Tumore, z.B. Tumore in Hohlorganen, behandelt werden. Hierbei kann die zur Freisetzung der Liposomeninhaltsstoffe erforderliche Erwärmung z.B. durch Spülen mit warmem Wasser erzielt werden. Beispiele hierfür sind HNO-Tumore, Lymphknotentumore, Lungentumore, Peritonealkarzinome, Pleurakarzinome, Ösophaguskarzinome, Magenkarzinome und Harnblasenkarzinome.

Erfindungsgemäß am meisten bevorzugt wird ein Harnblasentumor bzw. ein Harnblasenkarzinom behandelt. Es können dabei sowohl das Carcinoma in situ als auch das Stadium T1 sowie andere Stadien, z.B. T2 oder T3, des Harnblasenkarzinoms behandelt werden.

Neben der Therapie von oberflächlichen Harnblasentumoren kann ein solches Verfahren auch für die Therapie von muskelinvasiven Tumoren eingesetzt werden, um einen Blasenerhalt zu gewährleisten. Der durch dieses Verfahren gegenüber einer intravesikalen Chemotherapieapplikation erreichte Vorteil in Bezug auf eine verbesserte Tumorkontrolle sowie eine erhöhte Rate an Blasenerhaltenden Behandlungen verbunden mit einer geringen systemischen Nebenwirkungsrate lässt erwarten, dass dieses Verfahren die konventionelle intravesikale Chemotherapieinstillation ablösen wird.

Die erfindungsgemäßen Liposomen umfassen vorzugsweise weiterhin einen Wirkstoff, insbesondere ein Zytostatikum. Neben der üblichen für die intravesikale Therapie eingesetzten Zytostatika Mitomycin C, Doxorubicin und Epirubicin können auch andere Zytostatika wie z.B. Gemcitabin, Trabectedin, etc. für diesen Ansatz verwendet werden. Weitere geeignete Zytostatika sind Platinderivate wie z.B. Cisplatin, Carboplatin oder Oxaliplatin. Am meisten bevorzugt als Wirkstoff ist Doxorubicin.

Die Erwärmung des betroffenen Organs, z.B. der Blase, ist technisch durch eine Vielzahl von Verfahren möglich. So kann neben einer einfachen Blasenspülung durch erwärmtes Wasser eine Erwärmung durch elektromagnetische Wellen, Ultraschall oder auch mit Laser-Techniken durchgeführt werden.

Stimuli-sensitive Nanocarriersysteme, insbesondere thermosensitive Liposomen, die mindestens (i) ein Phosphatidylcholin mit einer Hauptumwandlungstemperatur von 0 °C bis 80 °C und (ii) mindestens ein Phosphatidyloligoglycerin umfassen, weisen eine lange Halbwertszeit im Serum auf. Zusätzlich wird der Inhalt solcher Nanocarriersysteme, insbesondere Liposomen, bei Ausübung eines Stimulus, z.B. bei Überschreitung einer bestimmten Temperatur, rasch freigesetzt, insbesondere in weniger als 10 s.

Ein Bestandteil von erfindungsgemäß bevorzugten stimuli-sensitiven Nanocarriersystemen, insbesondere thermosensitiven Liposomen, ist ein Phosphatidylcholin. Das Phosphatidylcholin liegt vorzugsweise in einer Menge von mindestens 1 Gew.-%, mehr bevorzugt mindestens 10 Gew.-%, noch mehr bevorzugt mindestens 30 Gew.-% und noch mehr bevorzugt mindestens 50 Gew.-% und bis zu 99 Gew.-%, mehr bevorzugt bis zu 90 Gew.-% und besonders bevorzugt bis zu 80 Gew.-%, bezogen auf das Gesamtgewicht der stimuli-sensitiven Nanocarriersysteme, insbesondere der Liposomen, vor.

Durch Auswahl eines Phosphatidylcholins mit einer jeweils geeigneten Hauptumwandlungstemperatur lässt sich das stimuli-sensitive Nanocarriersystem, insbesondere das thermosensitive Liposom, maßschneidern. In Tabelle 1 sind Hauptumwandlungstemperaturen (T_{M}) von Phosphatidylcholinen angegeben, deren Hauptumwandlungstemperaturen im Bereich von 0 bis 80 °C liegen. Die Hauptumwandlungstemperaturen sind, wie aus der Tabelle erkennbar, abhängig von der Kettenlänge und der Verteilung über die Positionen 1 und 2 von Glycero-3-phosphocholin oder über die Positionen 1 und 3 von Glycero-2-phosphocholin.

**Tabelle 1**

| Phasenumwandlungstemperaturen (T_{M}) von Phospholipiden | |
|---|---|
| T_{M} | Phosphatidylcholin/Phosphatidyldiglycerin/Phosphatidyltriglycerin/Phosphatidyltetraglycerin |
| 5 °C | 1-Palmitoyl-2-oleoyl- |
| 7 °C | 1-Stearoyl-2-oleoyl- |
| 11 °C | 1-Palmitoyl-2-lauroyl- |
| 14 °C | 1-Behenoyl-2-oleoyl- |
| 17 °C | 1-Stearoyl-2-lauroyl- |
| 19 °C | 1,3-Dimyristoyl- |
| 23 °C | 1,2-Dimyristoyl- |
| 27 °C | 1-Palmitoyl-2-myristoyl- |
| 33 °C | 1-Stearoyl-2-myristoyl- |
| 37 °C | 1-Myristoyl-2-palmitoyl- |
| 39 °C | 1,3-Dipalmitoyl- |
| 41 °C | 1,2-Dipalmitoyl- |
| 42 °C | 1-Myristoyl-2-stearoyl- |
| 46 °C | 1-Stearoyl-3-myristoyl- |
| 48 °C | 1-Stearoyl-2-palmitoyl- |
| 52 °C | 1-Palmitoyl-2-stearoyl- |
| 53 °C | 1,3-Distearoyl- |
| 56 °C | 1,2-Distearoyl- |
| 66 °C | 1,2-Diarachinoyl- |
| 75 °C | 1,2-Dibehenoyl- |
| 80 °C | 1,2-Dilignoceroyl- |

Die in der Tabelle 1 aufgeführten Werte zeigen, dass durch Verwendung der angegebenen Fettsäuren mit ungerader Kettenlänge und geeigneter Verteilung über das Glyceringrundgerüst praktisch jede gewünschte Temperatur im angegebenen Bereich von 0 bis 80 °C eingestellt werden kann. Die Fettsäureketten und ihre Verteilung über die Positionen 1 und 2 des Glycerinmoleküls dominieren die physikalischen Eigenschaften der Phospholipide. Die Phasenumwandlungstemperatur ist unabhängig davon, ob es sich um das Phosphatidylcholin, das Phosphatidyldiglycerin, das Phosphatidyltriglycerin oder das Phosphatidyltetraglycerin handelt.

Bevorzugt umfasst das erfindungsgemäße stimuli-sensitive Nanocarriersystem, insbesondere das thermosensitive Liposom, ein Phosphatidylcholin der Formel (I), 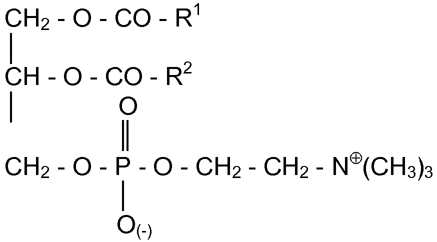 worin R¹ und R² jeweils unabhängig einen Kohlenwasserstoffrest mit 12 bis 24 C-Atomen darstellen.

R¹ und R² sind bevorzugt jeweils unabhängig voneinander ein gesättigter oder einfach oder mehrfach ungesättigter, vorzugsweise linearer Alkylrest, insbesondere ein gesättigter Alkylrest. R¹ und R² sind weiterhin bevorzugt jeweils unabhängig ein C14- bis C20-, insbesondere ein C14- bis C18-Rest.

Bevorzugt sind R¹ und R² jeweils unabhängig ein linearer gesättigter C12-bis C24-, insbesondere C12- bis C20-Alkylrest.

Am meisten bevorzugt sind R¹ und R² unabhängig ein linearer gesättigter C14-, C16- oder C-18-Alkylrest.

In einer weiteren Ausführungsform sind R¹ und R² bevorzugt jeweils unabhängig ein C13- bis C19-, insbesondere ein C13- bis C17-Rest. Am meisten bevorzugt sind R¹ und R² unabhängig ein linearer gesättigter C13-, C15- oder C17-Alkylrest.

Geeignete Phosphatidylcholine sind beispielsweise 1-Palmitoyl-2-oleoylglycero-3-phosphocholin, 1-Stearoyl-2-oleoyl-3-phosphocholin, 1-Palmitoyl-2-lauroylglycero-3-phosphocholin, 1-Behenoyl-2-oleoylglycero-3-phosphocholin, 1-Stearoyl-2-lauroylglycero-3-phosphocholin, 1,3-Dimyristoylglycero-2-phosphocholin, 1,2-Dimyristoylglycero-3-phosphocholin, 1-Palmitoyl-2-myristoylglycero-3-phosphocholin, 1-Stearoyl-2-myristoylglycero-3-phosphocholin, 1-Myristoyl-2-palmitoylglycero-3-phosphocholin, 1,3-Palmitoylglycero-2-phosphocholin, 1,2-Dipalmitoylglycero-3-phosphocholin, 1-Myristoyl-2-stearoylglycero-3-phosphocholin, 1-Stearoyl-3-myristoylglycero-2-phosphocholin, 1-Stearoyl-2-palmitoylglycero-3-phosphocholin, 1-Palmitoyl-2-stearoylglycero-3-phosphocholin, 1,3-Distearoylglycero-2-phosphocholin, 1,2-Distearoylglycero-3-phosphocholin, 1,2-Diarachinoylglycero-3-phosphocholin, 1,2-Dibehenoylglycero-3-phosphocholin und 1,2-Dilignoceroylglycero-3-phosphocholin.

Besonders bevorzugt weist das stimuli-sensitive Nanocarriersystem, insbesondere thermosensitive Liposom, mindestens ein Phosphatidylcholin mit einer Hauptumwandlungstemperatur im Bereich von 35 °C bis 42 °C, noch mehr bevorzugt im Bereich von 39 °C bis 41 °C auf. Stimuli-sensitive Nanocarriersysteme, insbesondere thermosensitive Liposomen, die solche Phosphatidylcholine enthalten, weisen eine Freisetzungstemperatur für den Inhalt auf, die es ermöglicht, dass die Liposomen im normalen Kreislauf (bei 37 °C) stabil sind und durch Wärmeeinwirkung, insbesondere lokale Wärmeeinwirkung, bei Temperaturen über 39 °C bis 41 °C ihren Inhalt freisetzen. Besonders bevorzugt sind stimuli-sensitive Nanocarriersysteme, insbesondere thermosensitive Liposomen, mit einer Freisetzungstemperatur von 40 bis 43 °C. Besonders bevorzugt weisen die erfindungsgemäßen stimuli-sensitiven Nanocarriersysteme, insbesondere thermosensitiven Liposomen, mindestens ein Phosphatidylcholin, ausgewählt aus 1,3-Dipalmitoyl-phosphatidylcholin und 1,2-Dipalmitoyl-phosphatidylcholin auf.

Bevorzugt umfasst das stimuli-sensitive Nanocarriersystem, insbesondere thermosensitive Liposom, ein Phosphatidylcholin der Formel (I) in natürlicher Konfiguration: 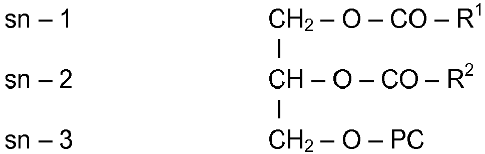 worin
- R¹, R²: wie oben definiert ist,
- sn: = stereospezifische Nummerierung ist
- PC: = Phosphocholin
- T_{M}: = Phasenumwandlungstemperatur

| | |
|---|---|
| Konfiguration: | natürlich |
| Bezeichnung: | 1.2-Diacyl-sn-glycero-3-phosphocholin |

Für eine therapeutische Anwendung bevorzugte Phosphatidylcholine sind solche, die eine Phasenumwandlungstemperatur um 40° C haben. Durch Zugabe von Phosphatidylcholinen mit T_{M} < 40° C oder durch Zugabe von Phosphatidylcholinen mit T_{M} > 40° C kann die T_{M} des stimuli-sensitiven Nanocarriersystems, insbesondere Liposoms, auf die gewünschte Temperatur eingestellt werden. Es hat sich dabei gezeigt, dass Unterschiede in der Alkylkettenlänge von > 4 CH₂-Gruppen zu Phasenseparationen führen und deshalb vermieden werden sollten.

Für andere Anwendungen, die nicht auf Stabilität bei etwa 37° C und Wirkstoff-Freisetzung bei 42° C angewiesen sind können Phospholipide eingesetzt werden, die andere T_{M} von < 37° C oder > 45° C besitzen.

Als wesentlichen Bestandteil enthalten die erfindungsgemäßen stimuli-sensitiven Nanocarriersysteme, insbesondere thermosensitiven Liposomen, mindestens ein Phosphatidyloligoglycerin.

Phosphatidyloligoglycerine umfassen Oligoglycerineinheiten, insbesondere Einheiten der Formel (III)

-[- O - CH₂ - CHOH - CH₂ -]ₙ- OH

worin n eine ganze Zahl von 2 bis 50, insbesondere eine ganze Zahl von 2 bis 10 und am meisten bevorzugt 2 oder 3 darstellt.

Erfindungsgemäß umfasst das stimuli-sensitive Nanocarriersystem, insbesondere thermosensitive Liposom, ein stereospezifisches Phosphatdidyloligoglycerin der Formel (IIa) 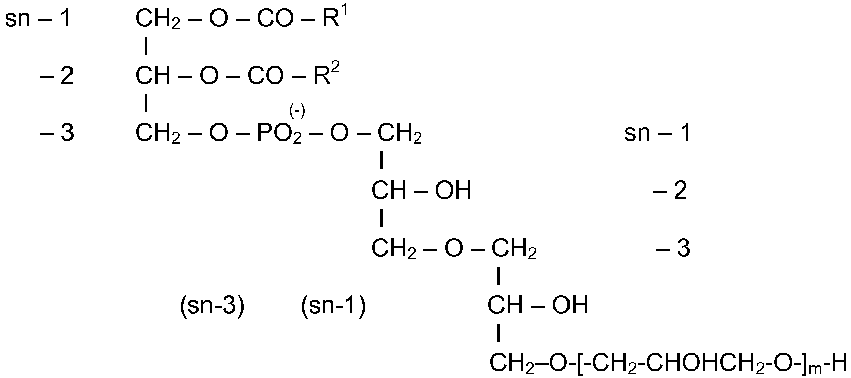 worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.

m ist vorzugsweise 0 bis 8 und am meisten bevorzugt 0 oder 1.

Besonders bevorzugt ist ein stereospezifisches Phosphatidyldiglycerin

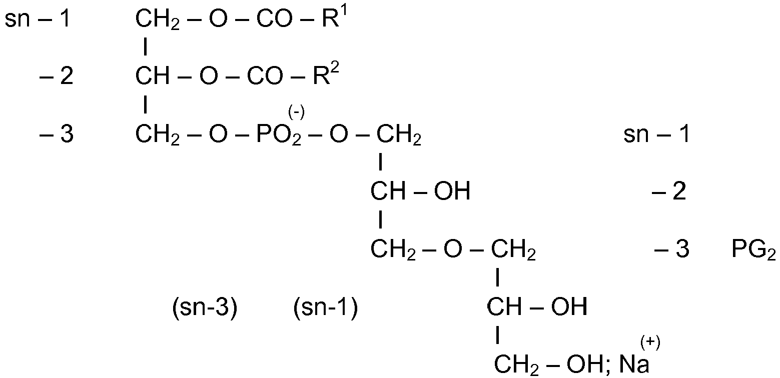

| | |
|---|---|
| Konfiguration: | natürlich |
| Bezeichnung: | 1.2-Diacyl-sn-3-glycero-phospho-sn-1-diglycerin; Natriumsalz (entsprechend auch Oligoglycerine, insbesondere tri- oder tetraglycerine) |
| | R₁, R₂, Fettsäuren; sn, stereospezifische Nummerierung; |
| | PG₂, Phosphodiglycerin |

worin R¹ und R² jeweils unabhängig einen Kohlenwasserstoffrest mit 12 bis 24 C-Atomen darstellen.

R¹ und R² sind bevorzugt jeweils unabhängig voneinander ein vorzugsweise linearer gesättigter oder einfach oder mehrfach ungesättigter Alkylrest, insbesondere ein gesättigter Alkylrest. R¹ und R² sind weiterhin bevorzugt jeweils unabhängig ein C14- bis C20-, insbesondere ein C14- bis C18-Rest.

Bevorzugt sind R¹ und R² jeweils unabhängig ein linearer gesättigter C12-bis C24-, insbesondere C12- bis C20-Alkylrest.

Am meisten bevorzugt sind R¹ und R² unabhängig ein linearer gesättigter C14-, C16- oder C-18-Alkylrest.

In einer weiteren Ausführungsform sind R¹ und R² bevorzugt jeweils unabhängig ein C13- bis C19-, insbesondere ein C13- bis C17-Rest. Am meisten bevorzugt sind R¹ und R² unabhängig ein linearer gesättigter C13-, C15- oder C17-Alkylrest.

Besonders bevorzugt umfassen die stimuli-sensitiven Nanocarriersysteme, insbesondere thermosensitiven Liposomen, wenigstens ein Phosphatidyldiglycerin oder/und wenigstens ein Phosphatidyltriglycerin. Es wurde festgestellt, dass solche stimuli-sensitiven Nanocarriersysteme, insbesondere Liposomen, umfassend Diglycerine und/oder Triglycerine, ein besonders günstiges Freisetzungsverhalten zeigen.

Erfindungsgemäß bevorzugt sind stimuli-sensitive Nanocarriersysteme, insbesondere Liposomen, in denen jeweils nur ein einziges Isomer einer Phosphatidyloligoglycerin-Verbindung enthalten ist, z.B. ausschließlich 1.2 Dipalmitoylphosphooligoglycerin und kein 1.3 Dipalmitoylphosphooligoglycerin.

Am meisten bevorzugt ist 1.2-Dipalmitoyl-sn-3-glycero-phospho-sn-1-diglycerin.

Für die physikalischen Eigenschaften der stimuli-sensitiven Nanocarriersysteme, insbesondere Liposomen, ist die Konfiguration der Phospholipide ohne Bedeutung, d. h. die physikalischen Eigenschaften ändern sich nicht, selbst wenn Racemate oder 1.3-Diacylglycerine in der Struktur vorkommen. Sie sind aber aus anderen Gründen weniger bevorzugt, denn sie können durch Phospholipasen nicht oder nur eingeschränkt abgebaut werden. Das gilt in besonderem Maße für die Phospholipasen A, B, C und D, die für den Abbau der Phospholipide verantwortlich sind. Es wurde bisher überhaupt nicht untersucht, ob z. B. 1.3-Dipalmitoyl-glycero-2-phospholipide metabolisch abgebaut werden können. Mit anderen Worten, die Frage ist unbeantwortet, ob sich solche Phospholipide im Organismus anreichern, ja sogar toxisch sein können.

Vor einer klinischen Anwendung muss deshalb sicher gestellt sein, dass die eingesetzten Phospholipide eine definierte Struktur haben - definiert in Struktur, Fettsäurezusammensetzung und Konfiguration. Sonst müsste alternativ in Langzeitstudien (Tierversuche) gezeigt werden, dass nicht natürliche Phospholipide in Struktur und Konfiguration keine toxischen Eigenschaften besitzen. Denn eine klinische Anwendung, insbesondere in der Indikation Krebs bedeutet, dass die Therapie über einen längeren Zeitraum durchgeführt werden muss.

Deshalb ist es besonders bevorzugt, dass die eingesetzten Phospholipide in Struktur, Konfiguration und Fettsäurezusammensetzung einheitlich und natürlich sind. Bevorzugt werden ausschließlich 1.2-Diacyl-sn-glycero-3-phosphocholine und 1.2-Diacyl-sn-glycero-3-phospho-sn-1-di-, tri- oder tetraglycerine verwendet.

Dies bedeutet, dass die erfindungsgemäßen Nanocarriersysteme insbesondere keine 1.3-Diacyl-glycero-3-phosphocholine und keine 1.2-Diacyl-glycero-3-phospho-oligoglycerine (oligo ≙ 2 bis 50, insbesondere 2 bis 10 Glycerineinheiten) umfassen.

Erfindungsgemäß werden insbesondere stereospezifische Lipide bereitgestellt bzw. verwendet. Mit früheren Herstellungsverfahren, in denen als Ausgansprodukt für die Posphorylierung meist 1.2-Diacylglycerin, z. B. 1.2-Dipalmitoyl-glycerin verwendet wurde, konnten Phosphatidyl-oligo-Glycerine in sehr guter Qualität erhalten werden (Reinheit > 99 %). Allerdings hat sich dann gezeigt, dass die strukturelle Reinheit nur im Bereich von ∼ 90 % lag; d. h. während der Phosphorylierung oder zum Teil auch bei der Lagerung von 1.2-Diacyl-Glycerin, z.B. 1.2-Dipalmitoyl-Glycerin, ist eine Fettsäurewanderung von der 2-Position in die 3-Position erfolgt. Es ist 1.3-Diacyl-Glycerin, z.B. 1.3-Dipalmitoyl-Glycerin, entstanden, immerhin ein einem Ausmaß von etwa 10 %. Das durch Fettsäurewanderung gebildete Diacyl-Glycerin, z.B. 1.3-Dipalmitoyl-Glycerin, wird ebenfalls phosphoryliert und in 1.3-Dipalmitoyl-2-phospho-oligo-glycerin umgewandelt. Die physikalischen Eigenschaften des 1.2- oder 1.3-Derivats sind sehr ähnlich, so dass eine Reinigung durch Chromatographie möglich, aber aufwendig ist.

Die neuen Verfahren vermeiden die Verwendung von 1.2-Diacyl-Glycerin, z.B. von 1.2-Dipalmitoyl-Glycerin, mit Hilfe eines völlig neuen Schutzgruppensystems, das gezielt darauf ausgerichtet ist, Phosphatdidyloligo-Glycerine chemisch analytisch rein, strukturell rein und in natürlicher Konfiguration herzustellen. Die dazu notwendigen Bausteine werden hierin beschrieben.

Die neuen Synthesewege haben den Vorteil, dass die eingesetzten Bausteine unter den Reaktionsbedingungen absolut stabil sind. Es findet keine Zersetzung oder Wanderung von Substituenten im Molekül statt.

Ein weiterer Vorteil der neuen Verfahren ist aber auch, dass nicht von Beginn an die Fettsäurezusammensetzung über die eingesetzten Diacylglycerine festgelegt wird. Das bedeutet für jedes angestrebte Fettsäuremuster separate Synthese- und Phosporylierungs-Schritte.

In den neuen Verfahren entfällt weiterhin die separate und aufwendige Herstellung der 1.2-Dialcylglycerine, z. B. von 1.2-Dipalmitoyl-glycerin. Erst am Ende der Synthese werden die beiden freien Hydroxylgruppen aus dem Isopropyliden-Glycerinrest freigelegt. Jetzt kann durch einfache Acylierung, z. B. mit Palmitinsäurechlorid oder mit anderen Fettsäurechloriden, eine Vielzahl von Fettsäurederivaten hergestellt werden. Danach werden wie üblich die Benzyl-Schutzgruppen durch katalytische Hydrogenolyse in Gegenwart von PD-C entfernt. Nach Abspaltung der Methyl-Schutzgruppe mit Lithiumbromid wird das Endprodukt erhalten.

Die physikalischen Eigenschaften der erfindungsgemäßen stimuli-sensitiven Nanocarriersysteme, insbesondere Liposomen, werden durch die Fettsäurezusammensetzung festgelegt, die die punktgenaue Thermosensitivität garantiert. Dagegen erfordern Serumstabilität und biologischer Abbau der Trägersysteme eine definierte Konfiguration. Diese kann nur durch chemische Synthese, aber nicht durch Umesterung mit Phospholipase D erreicht werden.

Die eingesetzten Phosphatidyl-oligo-glycerine bewirken eine lange Zirkulationszeit der stimuli-sensitiven Nanocarriersysteme, insbesondere Liposomen, im Blut. Die Fettsäurezusammensetzung garantiert eine Freisetzung der Wirkstoffe bei einer bestimmten Temperatur und Serumstabilität. Weiterhin bevorzugt sind Moleküle, die einen normalen biologischen Abbau gewährleisten, d.h. diese Moleküle liegen bevorzugt in natürlicher Konfiguration vor und sind durch Phospholipasen abbaubar, z. B. durch die Phospholipasen A, B und C.

Bevorzugte Phosphatidyloligoglycerine, die diese Eigenschaften besitzen, sind in ihrer Konfiguration einheitlich und liegen in natürlicher Konfiguration vor (siehe allgemeine Formel IIa).

Die Menge an Phosphatidyloligoglycerin beträgt vorzugsweise mindestens 1 Gew.-%, mehr bevorzugt mindestens 10 Gew.-% und noch mehr bevorzugt mindestens 15 Gew.-%, und bis zu 70 Gew.-%, mehr bevorzugt bis zu 50 Gew.-% und noch mehr bevorzugt bis zu 30 Gew.-%, bezogen auf das Gesamtgewicht der Nanocarriersysteme, insbesondere der Liposomen.

Die erfindungsgemäßen Nanocarriersysteme, insbesondere Liposomen, können auch Phosphatidylglyceroglykole umfassen. Bevorzugt sind Phosphatidylglyceroglykole der Formel (IV) 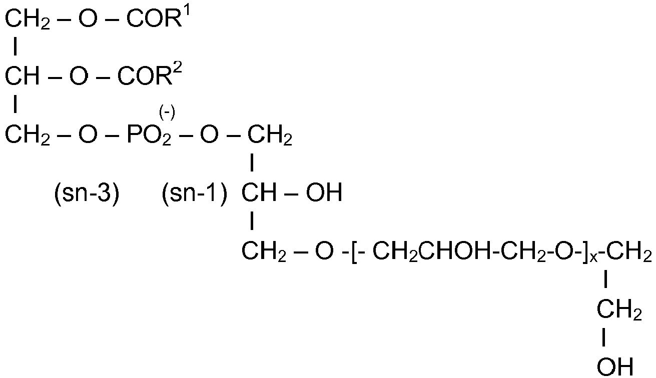 worin R¹ und R² jeweils unabhängig einen Kohlenwasserstoffrest mit 12 bis 24 C-Atomen darstellen und x eine ganze Zahl von 0 bis 50 ist.

R¹ und R² sind bevorzugt jeweils unabhängig voneinander ein vorzugsweise linearer gesättigter oder einfach oder mehrfach ungesättigter Alkylrest, insbesondere ein gesättigter Alkylrest. R¹ und R² sind weiterhin bevorzugt jeweils unabhängig ein C14- bis C20-, insbesondere ein C14- bis C18-Rest.

Bevorzugt sind R¹ und R² jeweils unabhängig ein linearer gesättigter C12-bis C24-, insbesondere C12- bis C20-Alkylrest.

Am meisten bevorzugt sind R¹ und R² unabhängig ein linearer gesättigter C14-, C16- oder C-18-Alkylrest.

In einer weiteren Ausführungsform sind R¹ und R² bevorzugt jeweils unabhängig ein C13- bis C19-, insbesondere ein C13- bis C17-Rest. Am meisten bevorzugt sind R¹ und R² unabhängig ein linearer gesättigter C13-, C15- oder C17-Alkylrest.

x ist vorzugsweise eine ganze Zahl von 0 bis 10, insbesondere 0, 1, 2 oder 3.

Die erfindungsgemäßen Nanocarriersysteme, insbesondere Liposomen, können auch ein Cardiolipin umfassen. Bevorzugt sind Cardiolipine der Formel (V) 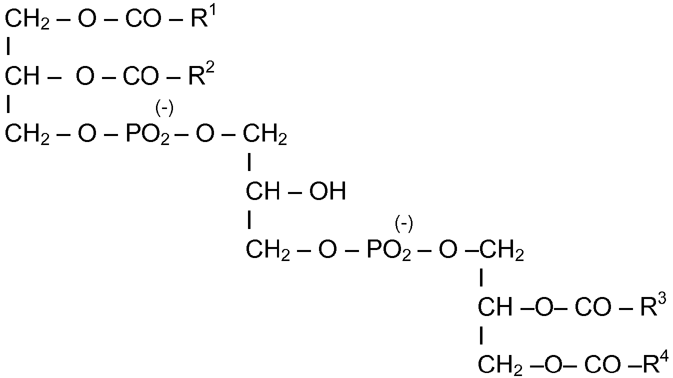 worin R¹, R², R³ und R⁴ jeweils unabhängig einen Kohlenwasserstoffrest mit 12 bis 24 C-Atomen darstellen und x eine ganze Zahl von 0 bis 50 ist.

R¹, R², R³ und R⁴ sind bevorzugt jeweils unabhängig voneinander ein vorzugsweise linearer gesättigter oder einfach oder mehrfach ungesättigter Alkylrest, insbesondere ein gesättigter Alkylrest. R¹, R², R³ und R⁴ sind weiterhin bevorzugt jeweils unabhängig ein C14- bis C20-, insbesondere ein C14- bis C18-Rest.

Bevorzugt sind R¹ und R² jeweils unabhängig ein linearer gesättigter C12-bis C24-, insbesondere C12- bis C20-Alkylrest.

Am meisten bevorzugt sind R¹, R², R³ und R⁴ unabhängig ein linearer gesättigter C14-, C16- oder C-18-Alkylrest.

In einer weiteren Ausführungsform sind R¹, R², R³ und R⁴ bevorzugt jeweils unabhängig ein C13- bis C-19-, insbesondere ein C13- bis C17-Rest. Am meisten bevorzugt sind R¹, R², R³ und R⁴ unabhängig ein linearer gesättigter C13-, C15- oder C17-Alkylrest.

Cardiolipine erhöhen die Zirkulationszeit von Nanocarriersystemen, insbesondere von Liposomen, im Blut.

Besonders bevorzugt sind stimuli-sensitive Nanocarriersysteme, insbesondere thermosensitive Liposomen, die im Serum bei 37° C stabil sind, d.h. keinen Wirkstoff freisetzen. Bei 42° C jedoch wird der Wirkstoff rasch in einem Zeitraum von < 15 sec freigesetzt. Die Freisetzung der liposomal eingeschlossenen Wirkstoffe kann z.B. gezielt in der Harnblasenwand erfolgen, wodurch der Wirkstoff, insbesondere ein Zytostatikum, unmittelbar an der gewünschten Stelle freigesetzt werden kann.

Für einen Einsatz dieser stimuli-sensitiven Nanocarriersysteme, insbesondere Liposomen, zur gezielten Therapie von lokalisierten Tumoren sind zunächst die physikalischen Eigenschaften der stimuli-sensitiven Nanocarriersysteme, insbesondere Liposomen, von entscheidender Bedeutung. Die Rahmenbedingungen sind vorgegeben durch die Körpertemperatur von etwa 37° C bei einem gesunden Menschen. Bei 36 bis 37° C sollten die mit Wirkstoff beladenen stimuli-sensitiven Nanocarriersysteme, insbesondere Liposomen, stabil sein. Bei einer lokalen Erhöhung der Temperatur im Tumorbereich auf 42° C sollte der Wirkstoff rasch, in < 15 sec freigesetzt werden. Diese stimuli-sensitiven Nanocarriersysteme, insbesondere Liposomen, können vorzugsweise mithilfe geeigneter Phospholipide hergestellt werden, die auf Fettsäureestern der Kettenlänge C₁₄ bis C₁₈ aufgebaut sind (Myristinsäure, Palmitinsäure, Stearinsäure: siehe dazu auch Tabelle 1). Diese Phospholipide durchlaufen im Bereich von etwa 40° C eine Phasenumwandlungstemperatur. Unter 40° C sind die lamellar angeordneten Phospholipide in der kristallinen Phase, darüber in der fluiden Phase. Im Umwandlungsbereich in einem engen Temperaturbereich zwischen 40 und 42° C werden die Wirkstoffe spontan freigesetzt.

Die Hauptumwandlungstemperatur für (1.2-Dipalmitoyl)-Phosphatidylcholin liegt bei etwa 41 °C. Die rein physikalischen Voraussetzungen für eine klinische Anwendung in Liposomen können also durch dieses Phosphatidylcholin erfüllt werden. Doch es fehlt für klinische Anwendungen die erforderliche Serumstabilität im Blutkreislauf von Mensch und Tier. (1-2-Dipalmitoyl)-Phosphatidylcholin alleine genügt deshalb nicht um die therapeutischen Ziele zu erreichen. Die vorgesehenen Therapieziele können also durch (Dipalmitoyl)-Phosphatidyl-cholin alleine nicht erreicht werden.

Durch den Einsatz von Phosphatidyloligoglycerinen ist es aber gelungen, stimuli-sensitive Nanocarriersysteme, insbesondere thermosensitive Liposomen, mit verlängerter Zirkulationszeit im Blut durch einfaches Zumischen von Phosphatidyldiglycerin oder Phosphatidyltriglycerin herzustellen. Ein einfaches Zumischen ist möglich, da Phosphatidylcholine, Phosphatidyldiglycerine oder Phosphatidyl-triglycerine bei gleicher Struktur und gleicher Fettsäureverteilung im Glycerinmolekül vergleichbare Phasenumwandlungstemperaturen besitzen, z.B. etwa 41 °C für die entsprechenden Dipalmitoylverbindungen.

Ein weiterer wichtiger Punkt ist, dass Phosphatidylcholine und Phosphatidyloligoglycerine ideale Mischungen ohne Phasenseparation ergeben, solange die Länge der Fettsäuren sich nicht um > 4 CH₂-Gruppen unterscheidet. Das ist eine wichtige Voraussetzung dafür, dass Phasenumwandlungstemperaturen zwischen 23° C (reines Dimyristoylphosphatidylcholin) und 41° C (reines Dipalmitoyl-phosphatidylcholin) nach Wunsch eingestellt werden können, ebenfalls zwischen 41° C und 56° C (reines Distearoylphosphatidylcholin). Entsprechend können in diesen Mischungen auch Phosphatidyloligoglycerine verwendet werden.

In einer Ausführungsform werden stimuli-sensitive Nanocarriersysteme, insbesondere thermosensitive Liposomen, mit einer Hauptphasenumwandlungstemperatur von etwa 40 °C bis 42 °C aus Mischungen aus (Dipalmitoyl)-phosphatidylcholin, (Distearoyl)-phosphatidylcholin, und (Dipalmitoyl)-Phosphatidyl-di- oder triglycerin, also Drei- Komponenten-Systeme gebildet. Das 1.2-Dipalmitoylphosphatidylcholin dient als Grundmatrix zur Einstellung der Phasenumwandlungstemperatur auf etwa 42 °C, das 1.2-Distearoylphosphatidylcholin führt zu einer leichten Anhebung der Phasenumwandlungstemperatur und (1.2-Dipalmitoyl)-Phosphatidyl-di- oder -triglycerin zur Herstellung der Serumstabilität und Stabilität in der Blutzirkulation.

Bevorzugt sind Systeme umfassend 40 - 60 Gew.-% 1.2-Dipalmitoylphosphatidylcholin, 15 - 25 Gew.-% 1.2-Distearoylphosphatidylcholin und 20 - 40 Gew.-% 1.2-Dipalmitoylphosphatidyldigylcerin. Die Bestandteile werden noch mehr bevorzugt jeweils in stereospezifischer Form eingesetzt.

In einer weiteren bevorzugten Ausführungsform genügt auch ein ZweiKomponenten-System aus (1.2-Dipalmitoyl)-Phosphatidylcholin und (1.2-Distearoyl)-Phosphatidyl-di- oder -triglycerin.

Durch den Einsatz erfindungsgemäßer stimuli-sensitiver Nanocarriersysteme, insbesondere thermosensitiver Liposomen, kann ein in den stimuli-sensitiven Nanocarriersystemen, insbesondere Liposomen, eingeschlossener Wirkstoff durch Ausüben eines Stimulus, insbesondere lokale Erwärmung der stimuli-sensitiven Nanocarriersysteme, insbesondere Liposomen, freigegeben werden. Deshalb eignen sich diese stimuli-sensitiven Nanocarriersysteme, insbesondere thermosensitiven Liposomen, besonders gut zur lokalen Therapie von Tumoren, insbesondere von Harnblasentumoren bzw. Harnblasenkarzinomen. Die Freisetzung des in den stimuli-sensitiven Nanocarriersystemen, insbesondere Liposomen, eingeschlossenen Wirkstoffs kann gezielt in der Harnblasenwand induziert werden, wodurch der Wirkstoff, insbesondere ein Zytostatikum, unmittelbar an der gewünschten Stelle freigesetzt werden kann. Diese Erfahrungen können auch zur Therapie von anderen soliden und lokalisierten Tumoren eingesetzt werden.

Die hier beschriebenen stimuli-sensitiven Nanocarriersysteme, insbesondere Liposomen, eignen sich besonders zur lokalen Freisetzung von Wirkstoffen durch Hyperthermie. Bevorzugt sind stimuli-sensitive Nanocarriersysteme, insbesondere Liposomen, die thermosensitiv sind und mindestens ein Phosphatidylcholin und mindestens ein Phosphatidyloligoglycerin mit Phasenumwandlungstermperaturen um jeweils 40 °C umfassen. Das bedeutet, dass als Fettsäureester vorzugsweise Myristinsäure-, Palmitinsäure- oder Stearinsäurester in Frage kommen. Natürlich können auch andere Phasenumwandlungstemperaturen über entsprechende Fettsäureester eingestellt werden (siehe dazu auch Tabelle 1).

Es wurde festgestellt, dass stimuli-sensitive Nanocarriersysteme, insbesondere thermosensitive Liposomen, die mindestens ein Phosphatidylcholin und mindestens ein Phosphatidyldiglycerin oder Phosphatidyltriglycerin mit Phasenumwandlungstemperaturen um 40° C aufweisen, lange Halbwertszeiten bei Normaltemperaturen um 36 bis 37° C im Blutkreislauf von Versuchstieren besitzen. Eingeschlossene Wirkstoffe werden erst freigesetzt, wenn lokal die Temperatur auf etwa 42 °C erhöht wird.

Das erfindungsgemäß beanspruchte stimuli-sensitive Nanocarriersystem, insbesondere thermosensitive Liposom, enthält vorzugsweise mindestens ein Phosphatidylcholin und mindestens ein Phosphatidyl-di, -tri- oder - tetraglycerin. Die Phasenumwandlungstemperatur richtet sich nach den Erfordernissen der Anwendung. Sie wird vorzugsweise auf etwa 42 °C für klinische Anwendungen eingestellt, einfach erreicht durch die möglichen Fettsäurekombinationen im Glycerinmolekül (siehe dazu auch Tabelle 1). Für andere Anwendungen können andere Phasenumwandlungstemperaturen unter 40° C interessant sein, zum Beispiel Temperaturen um < 30° C, die wieder über entsprechende Fettsäurekombinationen erreicht werden können (siehe Tabelle 1). Diese stimuli-sensitiven Nanocarriersysteme, insbesondere Liposomen, sind insofern interessant, weil sie beim direkten Einspritzen in den Tumorbereich ihren Wirkstoff sofort im Tumorgewebe freisetzen. Entsprechend sind auch stimuli-sensitive Nanocarriersysteme, insbesondere Liposomen, mit höheren Phasenumwandlungstemperaturen wichtig, da diese stimuli-sensitiven Nanocarriersysteme, insbesondere Liposomen, sehr stabil sind, den Wirkstoff nicht direkt ausschütten, aber den Wirkstoff langsam nach z. B. Aufnahme in Zellen freisetzen können, ohne dass ein Thermoeffekt notwendig ist. Es sind also stimuli-sensitive Nanocarriersysteme, insbesondere Liposomen, die auch noch bei 50° C oder mehr stabil sind.

Man kann drei Ausführungsformen dieser stimuli-sensitiven Nanocarriersysteme, insbesondere Liposomen, unterscheiden:
1) Nicht erfindungsgemäß
   Das aus Phospholipiden aufgebaute Liposom enthält mindestens ein Phosphatidylcholin und mindestens ein Phosphatidyloligoglycerin. Die Fettsäureketten sind so ausgewählt, dass eine Phasenumwandlungstemperatur von etwa 42° C erreicht wird. Die Position der Fettsäureketten im Glycerinmolekül und die Position des Phosphatrests sind beliebig - entscheidend ist die Phasenumwandlungstemperatur von etwa 42° C für einen therapeutischen Einsatz in vivo, die üblicherweise mit Fettsäure-estern der Kettenlänge C₁₄ bis C₁₈ (Myristinsäure, Palmitin- und Stearinsäure) erreicht werden kann.
2) Nicht erfindungsgemäß
   In diese Kategorie fallen Liposomen mit 1.2-Diacyl-sn-3-glycero-phospho-rac-oligoglycerine.
   Alle physikalischen Eigenschaften wie Phasenumwandlungstemperatur, ideales Mischungsverhalten ändern sich nicht. Allerdings kommen natürlicherweise diese Moleküle nicht vor und werden deshalb nicht oder nur langsam metabolisiert oder abgebaut.
3) Erfindungsgemäß
   In diese Kategorie fallen Liposomen aus Phospholipiden mit natürlicher Konfiguration: sn-glycero-3-phosphoräure-ester sn-1-phospho-di-, tri- oder tetraglycerine.
   Alle physikalischen Eigenschaften wie Phasenumwandlungstemperatur, ideales Mischungsverhalten sind unverändert. Die Konfiguration ist natürlich, d. h. der biologische Abbau der Moleküle ist gewährleistet.

Durch Auswahl eines Phosphatidylcholins mit einer jeweils geeigneten Hauptumwandlungstemperatur lässt sich das stimuli-sensitive Nanocarriersystem, insbesondere thermosensitive Liposom, maßschneidern. In Tabelle 1 sind Hauptumwandlungstemperaturen (T_{M}) von Phosphatidylcholinen angegeben, deren Hauptumwandlungstemperaturen im Bereich von 0 bis 80 °C liegen. Die Hauptumwandlungstemperaturen sind, wie aus der Tabelle erkennbar, abhängig von der Kettenlänge und der Verteilung über die Positionen 1 und 2 von Glycero-3-phosphocholin oder über die Positionen 1 und 3 von Glycero-2-phosphocholin.

Das Phosphatidylcholin liegt vorzugsweise in einer Menge von mindestens 10 Gew.-%, mehr bevorzugt in einer Menge von mindestens 30 Gew.-%, noch mehr bevorzugt in einer Menge von mindestens 50 Gew.-%, aber höchstens 90 Gew.-% in den stimuli-sensitiven Nanocarriersystemen, insbesondere Liposomen, vor.

Ganz allgemein kann die Phasenumwandlungstemperatur der Phosphatidylcholine über die Kettenlänge der Fettsäure-ester gesteuert werden wie die Tabelle 1 zeigt. Die Hauptumwandlungstemperatur (T_{M}) der Phosphatidylcholine kann maßgeschneidert auf den jeweiligen Bedarf eingestellt werden. Für klinische Anwendungen sind Temperaturen um 40° C von besonderer Bedeutung.

Neben den rein physikalischen Eigenschaften der stimuli-sensitiven Nanocarriersysteme, insbesondere Liposomen, d. h. Stabilität bei 37° C, aber Wirkstoff-Freisetzung bei 42° C in physiologischer Kochsalzlösung, können diese stimuli-sensitiven Nanocarriersysteme, insbesondere Liposomen, allerdings therapeutisch nur eingesetzt werden, wenn diese Voraussetzungen auch in Gegenwart von Serum und auch in Modellversuchen in Versuchsstieren vorhanden sind. Diese Stabilität kann grundsätzlich nicht erreicht werden, wenn Liposomen aus (Dipalmitoyl)-Phosphatidylcholin und (Dialmitoyl)-Phosphatidylglycerin eingesetzt werden. In diesem System zeigen Tierversuche, dass der Liposomeninhalt, der Wirkstoff in < einer Minute fast vollständig freigesetzt wird.

Die für einen klinischen Einsatz notwendigen Voraussetzungen, d. h. Stabilität bei 37° C, aber Wirkstoff-Freisetzung bei 42° C, konnte in Tierversuchen nur mit neuen negativen Ladungsträgern, den Phosphatidyloligoglycerinen, insbesondere den Phosphatidyldiglycerinen und Phosphatidyltriglycerinen erreicht werden.

Für den klinischen Einsatz muss aber ein weiterer Gesichtspunkt beachtet werden. Erfindungsgemäß wurden erstmals Phosphatidyldiglycerine und Phosphatidyltriglycerine mit Phosphorsäureestern, die eine sn-1-Verknüpfung und damit die natürlicherweise vorkommende Konfiguration aufweisen, hergestellt.

Ein weiterer Gegenstand der vorliegenden Anmeldung sind deshalb stereospezifische Phosphatidyloligoglycerine der Formel IIa

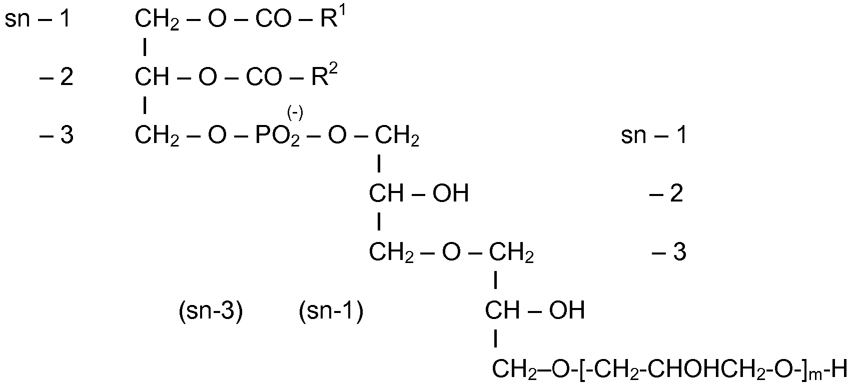

| | |
|---|---|
| Konfiguration: | natürlich |
| Bezeichnung: | 1.2-Diacyl-sn-3-glycero-phospho-sn-1-oligoglycerin; sn, stereospezifische Nummerierung; |

worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.

R¹ und R² sind bevorzugt jeweils unabhängig voneinander ein vorzugsweise linearer gesättigter oder einfach oder mehrfach ungesättigter Alkylrest, insbesondere ein gesättigter Alkylrest. R¹ und R² sind weiterhin bevorzugt jeweils unabhängig ein C14- bis C20-, insbesondere ein C14- bis C18-Rest.

Bevorzugt sind R¹ und R² jeweils unabhängig ein linearer gesättigter C12-bis C24-, insbesondere C12- bis C20-Alkylrest.

Am meisten bevorzugt sind R¹ und R² unabhängig ein linearer gesättigter C14-, C16- oder C-18-Alkylrest.

m ist vorzugsweise eine ganze Zahl von 0 bis10 und am meisten bevorzugt 0 oder 1.

Bei einer klinischen Anwendung, z.B. Tumortherapien, ist die physiologische Verträglichkeit von großer Bedeutung. Das stimuli-sensitive Nanocarriersystem, insbesondere Liposom, als Trägersystem sollte nach Abgabe des Wirkstoffs durch Phospholipasen leicht abgebaut werden können. Deshalb werden erfindungsgemäß als Phosphatidyl-diglycerine und Analoga ganz bevorzugt Moleküle verwendet, die eine Phosphorsäure-sn-1-Verknüpfung zu den Glycerinbausteinen besitzen. Der Vorteil besteht in einem raschen Abbau durch Phospholipasen.

Damit werden die grundsätzlichen Voraussetzungen für eine klinische Anwendung gegeben, insbesondere für eine Anwendung zur regionalen Therapie von Tumorerkrankungen, z.B. Blasentumor. Die Wirksamkeit dieser Therapien wurde bereits in Tierversuchen bestätigt.

Die erfindungsgemäßen stimuli-sensitiven Nanocarriersysteme, insbesondere thermosensitiven Liposomen, sind vorzugsweise frei von Cholesterin, da Cholesterin zu einer Verschmierung der Phasenumwandlungstemperatur führt und damit zu einem breiten thermischen Übergangsbereich. Insbesondere enthalten die erfindungsgemäßen stimuli-sensitiven Nanocarriersysteme, insbesondere thermosensitiven Liposomen, Cholesterin in einem Anteil von < 0,1 Gew.-%, mehr bevorzugt < 0,01 Gew.-%. Besonders bevorzugt enthalten die stimuli-sensitiven Nanocarriersysteme, insbesondere Liposomen, kein Cholesterin, sondern sind völlig frei von Cholesterin.

Gemäß einer bevorzugten Ausführungsform enthalten die erfindungsgemäßen stimuli-sensitiven Nanocarriersysteme, insbesondere Liposomen, zusätzlich geringere Mengen an Alkylphosphocholinen, vorzugsweise 10 bis 15 Gew.-%. Geeignete Substanzen sind z.B. Hexadecylphosphocholin, Oleylphosphocholin sowie Etherlysolecithine. Bei den Etherlysolecithinen kann die Hydroxylgruppe in Position 2 des Glycerins methyliert oder frei vorliegen. Bei dieser Ausführungsform gelingt es, die Freisetzung der im Liposom eingeschlossenen Substanzen von etwa 70 % ohne den Gehalt an Alkylphosphocholin auf praktisch 100 % zu erhöhen, was auf eine Beschleunigung der stimuli-sensitiven Nanocarriersysteme, insbesondere Liposomenöffnung, zurückzuführen ist. Des Weiteren weisen die Alkylphosphocholine einen antitumoralen Effekt durch temperaturabhängige Freisetzung aus den stimuli-sensitiven Nanocarriersystemen, insbesondere Liposomen, auf.

Die erfindungsgemäßen stimuli-sensitiven Nanocarriersysteme, insbesondere thermosensitiven Liposomen, enthalten weiterhin vorzugsweise einen Wirkstoff, insbesondere einen zur Behandlung von Harnblasentumoren geeigneten Wirkstoff. Bevorzugt enthalten die stimuli-sensitiven Nanocarriersysteme, insbesondere Liposomen, ein Zytostatikum, insbesondere ein Zytostatikum ausgewählt aus der Gruppe bestehend aus Mitomycin C, Doxorubicin, Epirubicin, Gemcitabin, Trabectedin, Cisplatin, Carboplatin und Oxaliplatin.

Die Thermosensitivität der erfindungsgemäßen Liposomen wird durch die Phasenumwandlung der Phosphatidylcholine innerhalb der Liposomenmembran bedingt. Wird die Phasenumwandlungstemperatur durchlaufen, so kommt es zu einer kurzzeitigen Membraninstabilität und dadurch zu einer Freisetzung des Liposomeninhalts. Dieser Effekt wird erfindungsgemäß zur Behandlung von Harnblasentumoren genutzt. Dabei wird der Harnblasentumor, beispielsweise im Rahmen einer regionalen Hyperthermie, regional spezifisch erwärmt. Dabei steigt die Temperatur im Tumor über die Grenztemperatur zur Freisetzung des Liposomeninhalts an. Der Liposomeninhalt wird dann spezifisch und nahezu ausschließlich im Tumor freigesetzt, sodass die Wirkstoffe effektiv zur Behandlung des Tumors eingesetzt werden können.

Die Erfindung betrifft deshalb weiterhin ein thermosensitives Liposom wie hierin beschrieben in Kombination mit Hyperthermie oder/und Ultraschall. Die Erwärmung kann dabei durch eine Vielzahl von Verfahren durchgeführt werden, wie beispielsweise eine einfache Blasenspülung mit warmem Wasser, eine Erwärmung durch elektromagnetische Wellen, Ultraschall oder Laser.

Die Herstellung der erfindungsgemäßen stimuli-sensitiven Nanocarriersysteme, insbesondere thermolabilen Liposomen, erfolgt in üblicher Weise durch Auflösen der Lipide, z.B. in Chloroform oder Chloroform/Wasser/Isopropanol, Abziehen des Lösungsmittels, zweckmäßig im Vakuum im Rotationsverdampfer, Tempern der Lipide mit wässrigen Lösungen der einzukapselnden Inhaltsstoffe bei Temperaturen, die über der Phasenumwandlungstemperatur liegen. Die Dauer dieser Temperungsbehandlung beträgt zweckmäßig 30 bis 60 Minuten, kann jedoch aber auch kürzer oder länger sein. Durch mehrfach wiederholte Einfrier-Auftau-Vorgänge, beispielsweise 2- bis 5-faches Einfrieren und wieder Auftauen, erfolgt eine Homogenisierung. Schließlich wird die erhaltene Lipidsuspension durch eine Membran definierter Porengröße bei einer Temperatur über der Phasenumwandlungstemperatur extrudiert, um die angestrebte stimuli-sensitive Nanocarriersystemgröße, insbesondere Liposomengröße, zu erreichen. Als Membran eignen sich beispielsweise Polycarbonatmembranen definierter Porengröße, wie 100 bis 200 nm. Schließlich kann gegebenenfalls nicht eingekapselter Inhaltsstoff abgetrennt werden, beispielsweise durch Säulenchromatographie oder dgl.

Die Freisetzung von Wirkstoffen bei der lokoregionären Therapie ist nicht auf die Behandlung von Harnblasentumoren limitiert.

Die Erfindung umfasst deshalb auch ein stimuli-sensitives Nanocarriersystem, insbesondere thermosensitives Liposom, zur Verwendung zur Behandlung von anderen Tumoren, insbesondere Weichteilsarkom, Osteosarkom, Blasenkarzinom (muskelinvasiv, *muscle invasive bladder cancer* [MIBC] und nicht-muskelinvasiv, *non-muscle invasive bladder cancer* [NMIBC]), Ovarialkarzinom, Magenkarzinom, Mammakarzinom (v.a. triple negatives Mamma-Ca, [TNBC]), Hepatozelluläres Karzinom, Uteruskarzinom, Schilddrüsenkarzinom, Kopf-Hals-Tumoren, Prostatakarzinom, Chordom, Desmoidtumor, Glioblastom und andere Tumorerkrankungen mit vorzugsweise lokoregionärer Ausbreitung.

Solche Nanocarriersysteme umfassen vorzugsweise einen zur Behandlung des jeweiligen Tumors geeigneten Wirkstoff, der dann stimuli-sensitiv im oder in der Nähe des Tumors freigesetzt wird.

Geeignete Wirkstoffe zur Behandlung von Tumoren sind beispielsweise Anthrazykline (z.B. Doxorubicin, Epirubicin), Oxazaphosphorine (z.B. Hydroxyifosfamid), Platinanaloga (Cisplatin, Oxaliplatin, Carboplatin), Gemcitabin, 5-Fluoruracil, Paclitaxel, Docetaxel, Etoposid, Topotecan, Vincristin, Irinotecan, Methotrexat, Bleomycin, Tyrosinkinaseinhibitoren, small molecules, DNA-Therapeutika oder Radiosensitizer (in Verbindung mit Strahlentherapie).

Weiterhin können die erfindungsgemäßen stimuli-sensitiven Nanocarriersysteme zur Behandlung von Infektionserkrankungen eingesetzt werden, insbesondere von Infektionserkrankungen, die durch Bakterien, Viren, Pilze oder/und Parasiten hervorgerufen sind.

Bevorzugt ist die Behandlung von Infektionen von medizinischen Implantaten, insbesondere orthopädischer Prothesen, die Behandlung von lokalisierten Infektionen, insbesondere Infektionen der tiefen Weichgewebe oder/und des Knochens, und/oder die Therapie multiresistenter Erreger.

Hierzu umfassen die stimuli-sensitiven Nanocarriersysteme, insbesondere thermosensitiven Liposomen vorzugsweise einen Wirkstoff, insbesondere ausgewählt aus Antibiotika, Virostatika, Fungiziden und antiparasitär wirkenden Arzneistoffen. Besonders bevorzugt ist der Wirkstoff ausgewählt ist aus

Antibiotika, insbesondere β-Lactame, Glykopeptide, Polyketide, Aminoglycosid-Antibiotika, Polypeptid-Antibiotika, Chinolone, Sulfonamide (z.B. Linezolid, Flucloxacillin, Cefazolin, Clindamycin, Vancomycin, Teicoplanin, Rifampicin Ampicillin, Ceftazidim, Ceftriaxon, Cefepim, Piperacillin, Fluorchinolone, Metronidazol, Amikacin, etc.) oder/und Virostatika, insbesondere Entry-Inhibitoren, Penetrations-Inhibitoren, DNA-Polymerase-Inhibitoren, DNA/RNA-Polymerase-Inhibitoren, Reverse Transkriptase-Inhibitoren, Inosinmonophosphat-Dehydrogenase-Hemmer, Proteaseinhibitoren, Integrase-Inhibitoren, Helikase-Primase-Inhibitoren, Cyclophilin-Inhibitoren, Maturations-Inhibitoren, Terminase-Inhibitoren, Neuraminidase-Inhibitoren, etc. oder/und
Fungiziden, insbesondere Azole (Benzimidazole ("MBC"), Triazole, Imidazole), Morpholine, Strobilurine, Chinoline, Anilino-Pyrimidine, Oxazolidin-dione, Carbonsäureamide, etc.

Die erfindungsgemäßen stimuli-sensitiven Nanocarriersysteme, insbesondere thermosensitives Liposomen, können weiterhin zur Behandlung von Erkrankungen des Auges, insbesondere zur Behandlung von entzündlichen, degenerativen, infektiösen und/oder neoplastischen Erkrankungen des Auges, Wundheilungsstörungen oder/und Glaukom eingesetzt werden.

Die stimuli-sensitiven Nanocarriersysteme, insbesondere thermosensitive Liposomen, können in einer weiteren bevorzugten Ausführungsform auch zur Behandlung von Autoimmunerkrankungen, insbesondere zur Behandlung von rheumatoider Arthritis oder/und chronisch entzündlichen Darmerkrankungen verwendet werden. Dazu umfassen die stimuli-sensitiven Nanocarriersysteme, insbesondere thermosensitive Liposomen, vorzugsweise weiterhin einen Wirkstoff, insbesondere ein Steroid, TNF-α oder/und Immunsuppressiva.

Die Erfindung stellt auch ein stimuli-sensitives Nanocarriersystem, insbesondere thermosensitives Liposom, zur Verwendung zur Diagnose bereit, insbesondere zur nicht-invasiven Temperaturmessung mit MR-Kontrastmittel und MR-Bildgebung. Ein solches stimuli-sensitives Nanocarriersystem, insbesondere thermosensitives Liposom, umfasst vorzugsweise als Wirkstoff ein CT- oder MRT-Kontrastmittel, vorzugsweise ausgewählt aus jodhaltigen Kontrastmitteln oder Gadolinium-Chelaten.

Die erfindungsgemäßen stimuli-sensitiven Nanocarriersysteme, insbesondere thermosensitive Liposomen, können weiterhin zur Behandlung von degenerativen Erkrankungen verwendet werden, insbesondere zur lokalisierten Freisetzung von entzündungshemmenden Mitteln, Schmerzmitteln oder/und Chondroprotektiva. Weitere Einsatzgebiete der stimuli-sensitiven Nanocarriersysteme, insbesondere thermosensitiven Liposome, sind die Behandlung von dementiellen Syndromen, Morbus Alzheimer oder/und fokalen neurologisch psychiatrischen Erkrankungen, insbesondere Epilepsie, sowie die Behandlung von Atherosklerose.

Zur Behandlung von Thrombosen enthalten die stimuli-sensitiven Nanocarriersysteme, insbesondere thermosensitive Liposome, vorzugsweise wenigstens einen Wirkstoff, ausgewählt aus Fibrinolytika, vorzugsweise Streptokinase, Urokinase oder/und Alteplase.

Die Erfindung wird durch die beigefügten Figuren und die folgenden Beispiele weiter erläutert.

### Beispiel 1

### Die Synthese von Phosphatidyl-oligoglycerinen

### Wichtige Bausteine

1) 3-Allyl-2-Benzyl-sn-G (Eigensynthese)
2) 1-Allyl-2-Benzyl-sn-G (Eigensynthese)
3) 1.2-Isopropyliden-sn-G(Handelsprodukt)
4) 2.3-Isopropyliden-sn-G(Handelsprodukt)

Mithilfe dieser Bausteine kann man die gewünschten Phosphatidyloligoglycerine entwickeln, mit natürlicher oder auch nicht natürlicher Konfiguration. Bisher wurden in diesem Bereich nur racemische Phosphatidyloligoglycerine hergestellt und in langzeitzirkulierenden Liposomen getestet.

### Bisher:

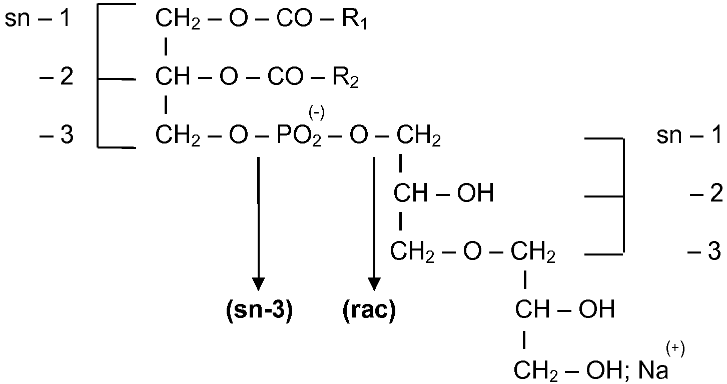

| | |
|---|---|
| Konfiguration: | racemisch (nicht natürlich) |
| Bezeichnung: | 1.2-Diacyl-sn-3-glycero-phospho-rac-diglycerin (entsprechend Tri- oder Tetraglycerine) |
| Erläuterungen: | R₁, R₂ - gesättigte Alkylreste; sn-stereospezifische Nummerierung; rac-racemische Verknüpfung; entsprechend auch Tri- oder Tetraglycerine |

### Erfindungsgemäß:

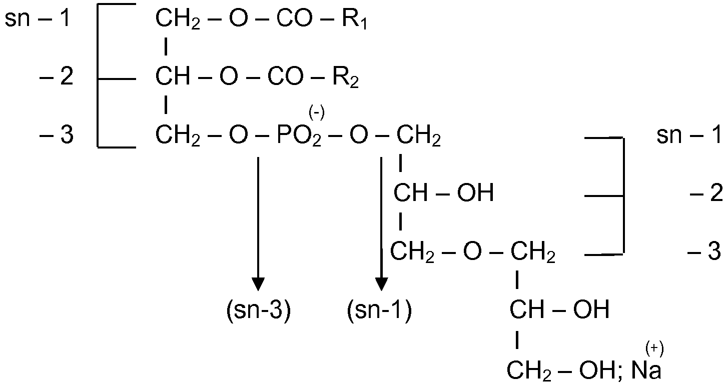

| | |
|---|---|
| Konfiguration: | natürlich |
| Bezeichnung: | 1.2-Diacyl-sn-3-glycero-phospho-sn-1-diglycerin (entsprechend auch tri- oder tetraglycerine) |
| Erläuterungen: | R₁, R₂-gesättigte Alkylreste; sn-stereospezifische Nummerierung; diglycerine-entsprechend auch tri- oder tetraglycerine |

Die Substanzen natürlicher Konfiguration zu den Phosphatestern, also (sn-3)-Verknüpfung von Diacylglycerin, dagegen (sn-1)-Verknüpfung der Di- Tri- oder Tetraglycerine werden hier erstmals beschrieben. Sie können aus Lecithinen durch Umesterung mit Phospholipase D in Gegenwart von Glycerin nicht erhalten werden, sondern nur durch gezielte Synthese. Die Substanzen liegen in natürlicher Konfiguration, also (sn-3)- und (sn-1)-Verknüpfung vor.

Auch bei der Herstellung der weiteren Substanzen wie z. B.
Glycero-glykole
Glycero-glycerine (verzweigt)
Glycero-glycero-glykole
wurden Synthesewege entwickelt, die gewährleisten, dass diese Substanzen einen Glycerinrest enthalten, der eine (sn-1)-Verknüpfung des Phosphatesters zum Phosphatidyl-oligoglycerin aufweist. Diese Substanzen enthalten in der Struktur also mindestens ein Glycerinmolekül mit freier OH-Gruppe, die eine (sn-1)-Verknüpfung ermöglicht. Die Substanzen sind neu, weil auch hier eine (sn-3)-Verknüpfung des Diacylglycerinphosphorsäureesters sowie eine (sn-1)-Verknüpfung zum Glycerolglykol möglich ist. Außerdem ist hier auch die Struktur neu, weil endständig nicht Glycerin, sondern Glykol oder analoge stehen.

### Beispiel 2

### Beispiele für die Synthese von Phosphatidyl-oligoglycerinen mit einheitlicher und natürlicher Konfiguration

Verwendete Abkürzungen: P, Palmitinsäure; O, Ölsäure; S, Sterinsäure; L, Laurinsäure; B, Behensäure; M, Myristinsäure; A, Arachinsäure; Li, Linocerinsäure.

PC, Phosphocholin; PG₂, Phosphodiglycerin; PG₃, Phosphotriglycerin; PG₄, Phosphotetraglycerin.

### Diglycerine

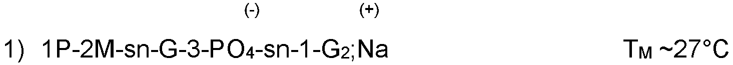

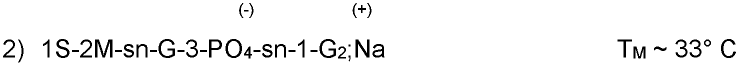

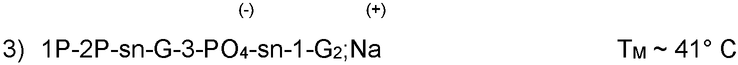

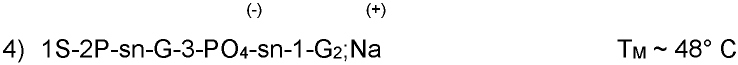

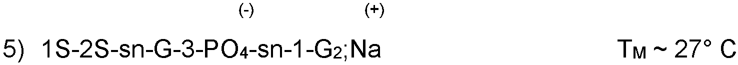

### Triglycerine

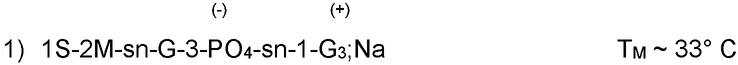

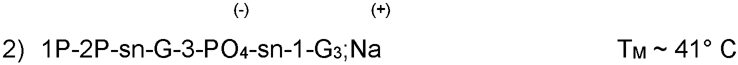

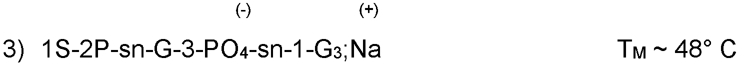

### Tetraglycerine

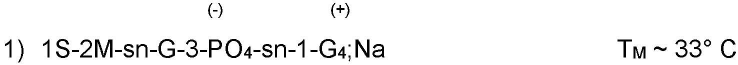

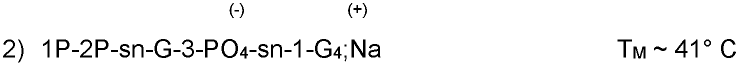

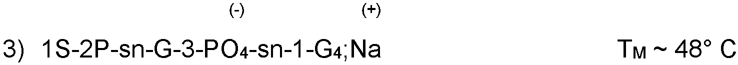

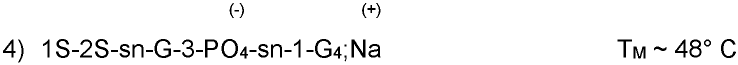

### Beispiel 3

### Beispiele für die Synthese von Phosphatidyl-sn-1-Glyceroverbindungen mit natürlicher Konfiguration

### (Neuheit durch Strukturvariation sowie durch einheitliche und natürliche Konfiguration)

### Glycero-Glykole (G-Gly)

### Struktur:

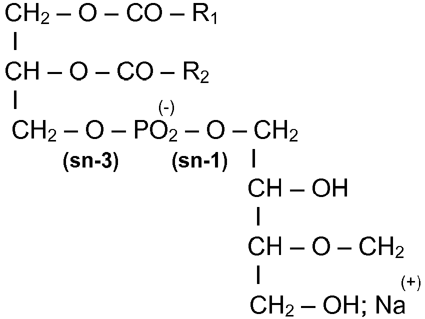

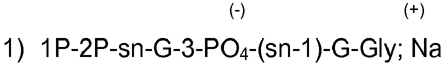

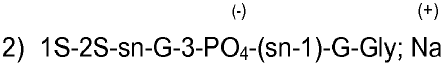

### Glycero-Glycerine (verzweigt)

### Struktur:

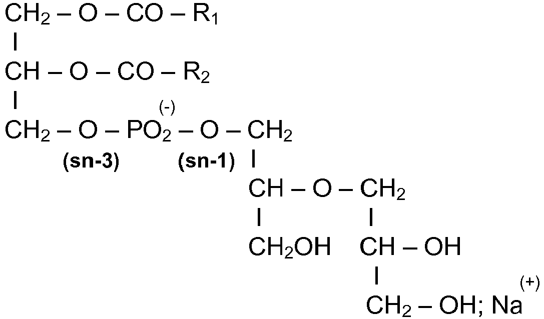

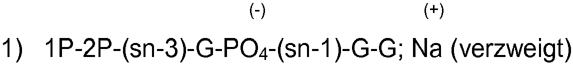

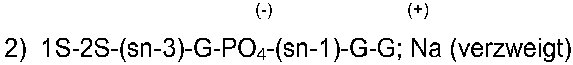

### Diglycero-Glykole (G₂-Gly)

### Struktur:

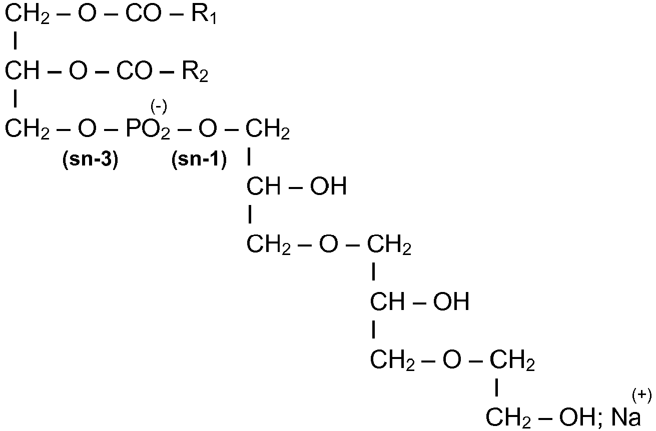

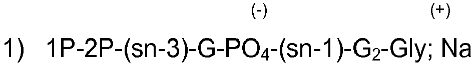

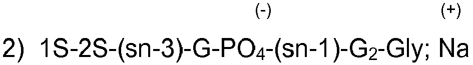

### Beispiel 4

### Verwendung von thermosensitiven Liposomen zur Behandlung des Harnblasenkarzinoms

Erfindungsgemäße thermosensitive Liposomen umfassend 30 mol-% Dipalmitoylphosphatidyldiglycerin oder Dipalmitoylphosphatidyltriglycerin wurden eingesetzt.

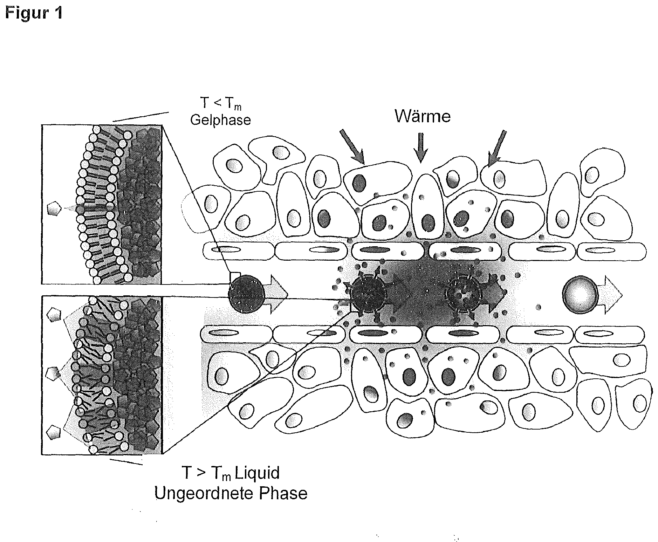

Figur 1 zeigt das Prinzip der intravaskulären Wirkstofffreisetzung aus thermosensitiven Liposomen.

Wirkstofffreisetzung tritt allgemein durch passiven Transfer über die Lipidmembran aufgrund eines Konzentrationsgradienten auf. Bei ihrer Phasenübergangstemperatur (Tₘ) gehen Phospholipide von einer festen Gelphase (L_{β}) in eine flüssige ungeordnete Phase (L_{α}) über. Die L_{α}-Phase ist durch eine größere Permeabilität im Vergleich zur L_{β}-Phase charakterisiert. Bei Temperaturen nahe der Phasenübergangstemperatur Tₘ ist die Permeabilität am größten aufgrund der Koexistenz von Membranbereichen, in denen beide Phasen auftreten, wodurch Grenzbereiche mit Packungsdefekten gebildet werden. Die erfindungsgemäßen thermosensitiven Liposomen setzen ihren Inhalt in den Blutstrom frei, wenn sie erwärmtes Gewebe passieren. Dadurch wird eine hohe Wirkstofffreisetzungsrate bereitgestellt.

Es wurden in vivo-Experimente in weiblichen F344-Ratten mit einem Gewicht von 170 bis 200 g durchgeführt, die einen orthotopischen Blasenkrebs durch Inokulierung mit AY27-Zellen entwickelten. Pharmakokinetiken und Akkumulation von Doxorubicin (Dox) wurden durch HPLC-Messungen evaluiert. Fokales Tumorwachstum wurde durch chemische Präkonditionierung der Blasenwand, gefolgt durch AY27-Zelllinstillation initiiert. Tumorwachstum wurde durch Zystoskopie inspiziert. TSL(Dox) (thermosensitive Liposomen enthaltend Doxorubicin) oder freies Dox wurden intravenös (i.v.) injiziert oder intravesikal eingebracht. Temperaturerwärmung der Blase wurde durch warmes Wasser bewerkstelligt.

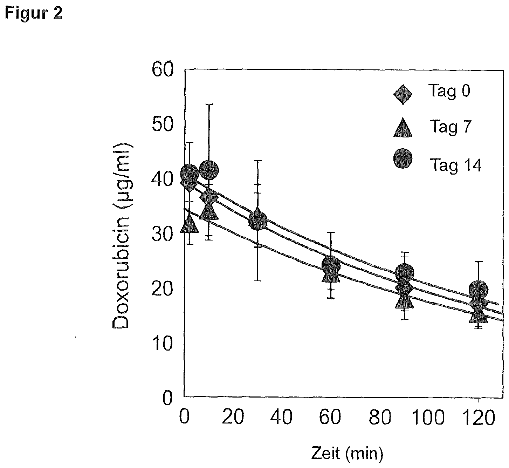

Figur 2 zeigt das pharmakokinetische Profil von TSL(Dox).

Eine TSL(Dox)-Applikation von 2 mg/kg wurde an Tag 0 mit einer Wiederholung nach 7 oder 14 Tagen in weiblichen F344-Ratten durchgeführt. TSL(Dox) zeigen eine hohe Stabilität über 120 Minuten.

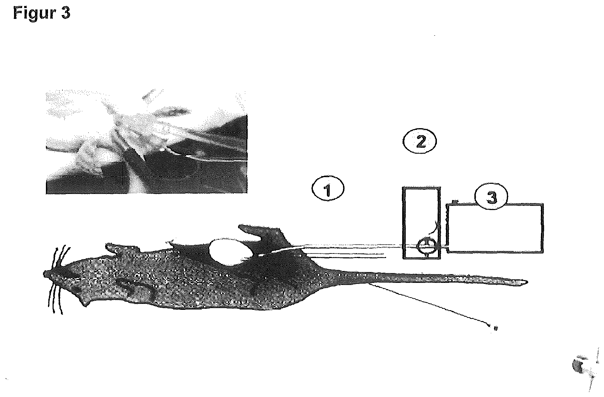

Figur 3 zeigt den experimentellen Aufbau des Ratten-Blasenkarzinom-Modells (modifiziert von Postius, Szelinyi; J. Pharmacol. Methods. 1983, 9: 53-61).

Die Ratte wird mit zwei Blasenkathetern (1) versehen. Eine Präzisionspumpe (2) transportiert erwärmtes Wasser vom Wasserbad (3) durch einen der Katheter in die Blase. Der andere Katheter dient zum Ausfluss des warmen Wassers. Wenn die Temperatur des ausfließenden Wassers 41 °C erreicht, werden Doxorubicin enthaltende thermosensitive Liposomen in die Schwanzvene injiziert und eine einstündige Hyperthermie-Behandlung gestartet.

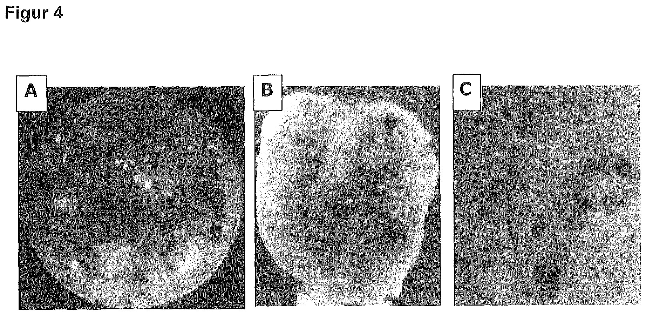

Figur 4 zeigt eine Tumormakroskopie sieben Tage nach Tumorzellinokulation.

Die Experimente wurden in weiblichen F344-Ratten mit einem Gewicht von 170 bis 200 g durchgeführt, die einen orthotopischen Blasenkrebs durch Inokulation mit AY-Zellen entwickelten. Pharmakokinetiken und Akkumulation von Doxorubicin (Dox) wurde durch HPLC-Messungen evaluiert. Fokaler Tumorwachstum wurde durch chemische Präkonditionierung der Blasenwand gefolgt von AY27-Zellinstillation initiiert. Multiple Tumorplaquebildungen auf der Blasenwand wurden mit Zytoskopie (A) und an einer entnommenen Blase (B und C) visualisiert.

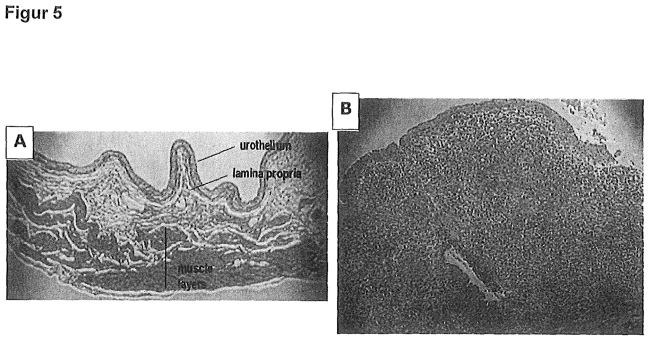

Figur 5 zeigt die Histologie einer Blase.

Figur (A) zeigt eine normal erscheinende Blase einer F344-Ratte in 10-facher Vergrößerung. Figur (B) zeigt einen pT1G3-Tumor sieben Tage nach Tumorzellinokulation ebenfalls in 10-facher Vergrößerung.

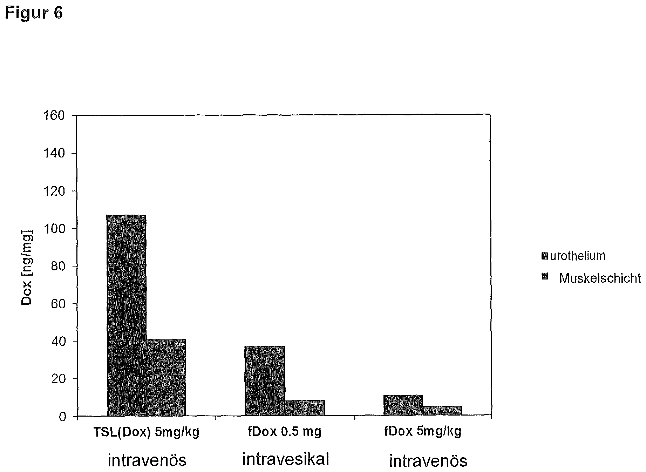

Figur 6 zeigt Gewebekonzentrationen von Dox in der Blasenwand von weiblichen F344-Ratten mit Blasentumor.

Die Abbildung zeigt die mittlere Dox-Konzentration im Urothelium und in der Muskelschicht nach einer einstündigen Behandlung mit intravenös verabreichtem TSL(Dox) und Hyperthermie, intravesikal verabreichtem freiem Dox (fDOX) und intravenös verabreichtem freiem Dox. Die Dox-Dosis für die i.v.-Behandlung betrug 5 mg/kg Körpergewicht, für die intravesikale Behandlung 0,5 mg/Ratte. Die Dox-Konzentration im Urothelialium war in allen Fällen, unabhängig von der Behandlungsprozedur, höher. Die Konzentration im Urothelium/in der Muskelschicht einer Tumor-tragenden Ratte, die mit TSL(Dox) + Hyperthermie behandelt wurde, war bis 3- bis 5-fach höher im Vergleich zu intravesikal verabreichtem freiem Dox.

### Beispiel 5

**Wichtige Bausteine für die Synthese von Phosphatidyl-diglycerinen und von Analoga**
A) (sn)-1.2-Isopropylidenglycerin
   ist ein Handelsprodukt mit freier (sn)-3-Hydroxylgruppe. Es kann direkt zur Phosphorylierung eingesetzt werden. Es entstehen Phosphorsäureester mit (sn)-3-Verküpfung: 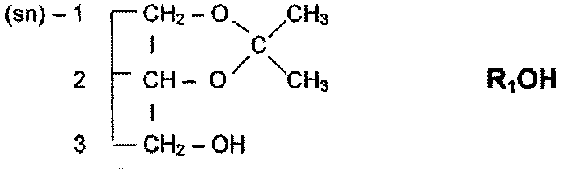
B) (sn)-3-Allylglycerin
   kann aus A durch Umsetzung mit Alkylchlorid und dann durch saure Abspaltung der Isopropyliden-Schutzgruppe erhalten werden. Es dient zum Aufbau von optisch reinen Glycerinderivaten, die eine freie Hydroxylgruppe in (sn)-1-Position besitzen.
C) (sn)-1 Trityl-2Benzyl-3-Allyl-Glycerin
   kann aus B durch Tritylierung in (sn)-1 und anschließender Benzylierung in (sn-2) erhalten werden.
D) (sn)-1 Trityl-2-Benzyl-3-Glycero-Glycerin
   kann aus C entwickelt werden über Epoxydation und Ringöffnung.
E) (sn)-2Benzyl-3Glycero-Dibenzyl-Glycerin
   kann aus D durch Dibenzylierung und anschließender Detritylierung hergestellt werden. Es entsteht ein wichtiger Baustein, der (sn)-1-Verknüpfung und damit die Herstellung von (sn)-1-Phosphorsäureestern ermöglicht. 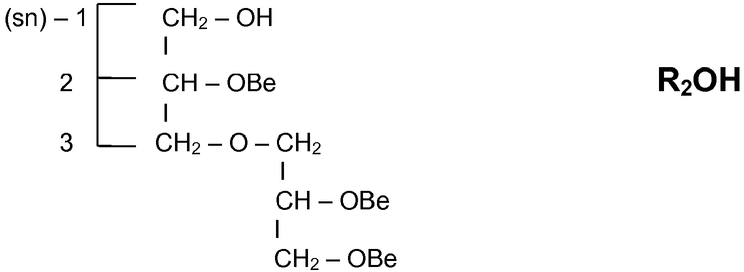
F) (sn)-1-OH-2-Benzyl-3-Glycero-Benzyl-Glykol
   kann aus D entwickelt werden: Vicinale Diolspaltung mit Perjodat und Reduktion des Aldehyds zum Alkohol mit Natriumborhydrid ergibt 1-Trityl-2-Benzyl-3-Glycero-Glykol. Benzylierung der freien Hydroxylgruppe sowie Detritylierung führt zur Freilegung der (sn)-1-Position, die wieder zur Herstellung von (sn)-1-Phosphorsäureestern verwendet werden kann.

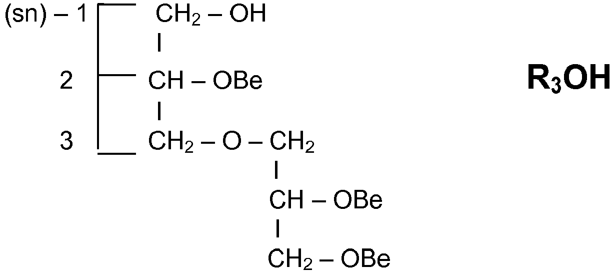

Entsprechend kann aus Triglycerinen mit der gleichen Schutzgruppenstrategie (sn)-1 OH-2 Benzyl-3 Glycero-2 Benzyl-Glycero-Benzyl-Glykol entwickelt werden: 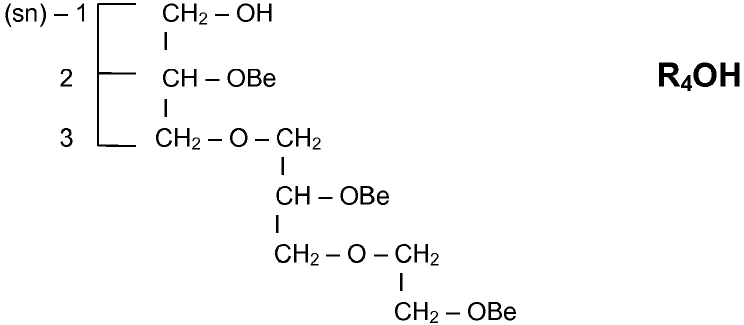 G) (sn)-1 Acetyl-2 Benzyl-Glycerin
diese Schutzgruppe wird erst eingesetzt zur Herstellung komplexer Phospholipide wie z. b. Kardiolipin, das in einem Molekül zwei Phosphorsäureester enthält. Die in dem Molekül vorhandenen Phosphatidylreste sind über eine Glycerinbrücke miteinander verbunden. Der obige Baustein erlaubt die Herstellung dieser Brücke sowie die Herstellung der beiden Phosphorsäureester in (sn)-3-Position, also ebenfalls in der natürlicherweise vorkommenden Konfiguration.
G kann aus dem Baustein C entwickelt werden: Detritylierung in (sn)-1- Position, Umlagerung von Allyl in Propenyl, Acetylierung der (sn)-1- Position und saure Abspaltung der Propenylschutzgruppe ergibt den Baustein G mit freier (sn)-3-Position:

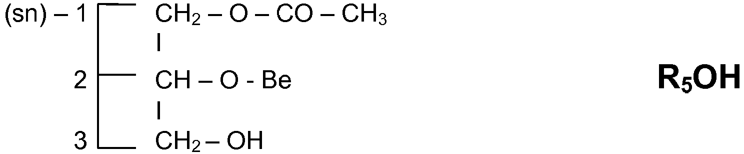

Für die Herstellung von Kardiolipinen und Analoga müssen besondere Maßnahmen ergriffen werden, da in diesen Molekülen zwei negative Ladungsträger, also zwei Phosphatreste pro Molekül vorhanden sind. Dazu setzt man den bereits phosphorylierten Rest R₅ nach Abspaltung der Acetylgruppe als Alkohol R₆ OH ein (siehe dazu Herstellung der Phosphorsäuretriester: Herstellung des Phosphorsäuretriesters aus R₅OH und Herstellung des Phosphorsäuretriesters aus R₆OH)

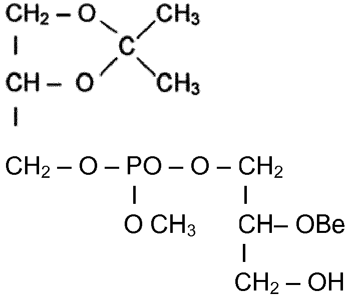

Setzt man R₆OH nochmals mit Phosphoroxychlorid um, so erhält man das Diphosphat. Beide Phosphorsäurereste sind dann über eine Glycerinbrücke miteinander verbunden.

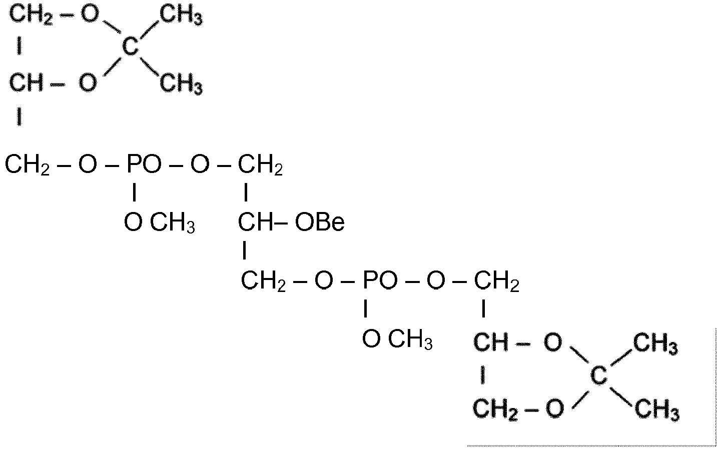

Am Ende der Synthese erfolgt dann die Freilegung der vier durch Isopropyliden geschützten Hydroxylgruppen. Danach folgt wie üblich Acylierung nach bekannten Verfahren Fettsäurechlorid,z.B. Palmitinsäurechlorid oder Palmitinsäure. Nach Abspaltung der Benzylether-Schutzgruppen durch Katalytische hydrogene Hydrogenolyse mit PD-C erhält man das Zielprodukt durch Abspaltung der Methylgruppe mit Lithiumbromid.

### 1-Acetvlpropandiol-(1.3)

kann ebenfalls als Brückenmolekül eingesetzt werden und führt dann zu den sogenannten Desoxy-Kardiolipinen - ebenso mit anderen endständigen Diolen wie Glykol, Butandiol-(1.4) usw. Diese Verbindungen sind in unserem Konzept, möglichst natürlicherweise vorkommende Phosphatidyl-Derivate einzusetzen, von geringerem Interesse.

### Beispiel 6

### Herstellung strategisch wichtiger Phosphorsäure-triester durch schrittweise Umsetzung von POCl₃ mit drei unterschiedlichen, primären Alkoholen

Die Herstellung definierter, positionsspezifischer und in der Konfiguration eindeutiger Phosphorsäuretriester ist intensiv untersucht worden (siehe dazu die Publikationen 1-10).
1) Eibl H. Synthesis of glycerophospholipids. Chem Phys Lipids. 1980 Jun;26(4):405-29.
2) Eibl H. Phospholipid synthesis. In: Liposomes: From physical structure to therapeutic applications. Ed Knight C G, Elsevier, Amsterdam 1981; 19-50
3) Eibl H, Kovatchev S. Preparation of phospholipids and their analogs by phospholipase D. Methods Enzymol. Ed Löwenstein J M. 1981;72:632-9. Academic Press, New York
4) Eibl H. Phospholipide als funktionelle Bausteine biologischer Membranen. Angew Chem. 1984 (259): 9188-9198
5) Eibl H. Phospholipids as functional constituents of biomembranes. Angew Chem Int. Ed Engl 1984 (23) 257-271
6) Eibl H. Phospholipid synthesis: Oxazaphospholanes and dioxaphospholanes as intermediates. Proc Natl Acad Sei USA 1978;75:4074-77
7) Eibl H, Woolley P. Synthesis of enantiomerically pure glyceryl esters and ethers. I. Methods employing the precursor 1,2-isopylidene-sn-glycerol. Chem Phys Lipids 1986 (41):53-63
8) Eibl H, Woolley P. Synthesis of enantiomerically pure glyceryl esters and ethers. II. Methods employing the precursor 3,4-isopropylidene-D-mannitol. Chem Phys Lipids. 1988 (47):47-53
9) Eibl H, Woolley P. A general synthetic method for enantiomerically pure ester and ether lysophospolipids. Chem Phys Lipid 1988 (47):63-68
10) Woolley P, Eibl H. Synthesis of enantiomerically pure phospholipids including phosphatidylserine and phosphatidylglycerol. Chem Phys Lipids 1988 (47): 55-62

Aus den hier beschriebenen Bausteinen können unterschiedliche Phosphatidyloligo-glycerine und entsprechend Phosphatidyl-glyceroglykole hergestellt werden. Besonders wichtig ist, dass die Fettsäurereste erst am Ende der Synthese aus einem zentralen Zwischenprodukt hergestellt werden, d.h. im Gegensatz zu früheren Synthesen werden die Fettsäurereste erst zum Schluss eingeführt. Das ist möglich durch die Herstellung eines zentralen Zwischenprodukts mit zwei vicinalen Hydroxylgruppen, z.B.

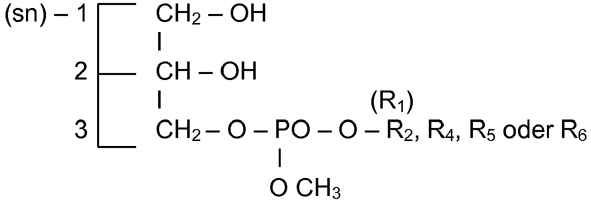

In der deutschen Patentanmeldung 196 05 833.3 vom 16. Februar 1996 (Erfinder: H. Eibl; Inhaber: Max-Planck-Gesellschaft) sind die Bedingungen für die schrittweise Veresterung von Phosphoroxychlorid mit primären Alkoholen diskutiert und exakt beschrieben. Im Unterschied zu den früheren Verfahren, in denen im ersten Schritt 1.2-Diacylglycerin, z. B. 1.2-Dipalmitoylglycerin verwendet wurde (Problem: Fettsäurewanderung bei der Reaktion oder bei der Lagerung von 1.2-Dipalmitoylglycerin, das vorher separat hergestellt werden musste) wird die Synthese eingeleitet mit (sn)-1.2-Isopropylidenglycerin mit freier sn-3-Position, das käuflich erworben werden kann. Das erspart die aufwendige, separate, Synthese von 1.2-Oiacylglycerin, z. B. von 1.2-Dipalmitoylglycerin und den damit oben genannten Problemen.

Nach diesen schlechten Erfahrungen mit 1.2-Dipalmitoyl-glycerin im ersten Phosphorylierungsschritt wurde die Synthese anders aufgebaut. Ziel war die Fettsäureester erst am Ende der Synthese über ein freigelegtes vicinales Diol einzuführen. Folgende Reihenfolge der Syntheseschritte ist bevorzugt:
**P₁** - erster Phosphorylierungsschritt mit Phosphoroxychlorid
   R₁ OH: (sn)-1.2-Isopropyliden-Glycerin
   (freie sn-3-Position)

   POCl₃ + R₁ OH → R₁ O - PO Cl₂
**P₂** - zweiter Phosphorylierungsschritt mit R₁ O - PO Cl₂
   R₂ OH oder entsprechend mit freier sn-1-Position
   R₃ OH; R₄OH; R₅OH; R₆OH 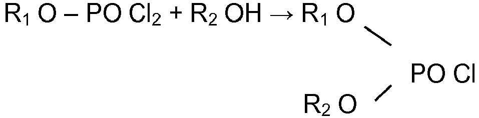
**P₃** - dritter Phosphorylierungsschritt mit

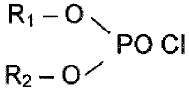

### Methanolyse mit CH₃ OH

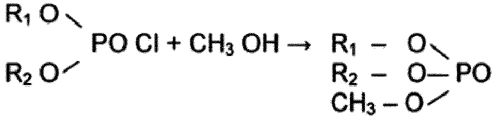

Weiter werden nach Phosphorylierung mit den benzylgeschützten Glycerinen die Phosphorsäurediester nicht direkt hydrolysiert, sondern durch Methanolyse in Phosphorsäure-triester umgewandelt. Nun kann die Freilegung des ricinalen Diols durch saure Abspaltung der Isopropylidenschutzgruppe erfolgen. Dann folgt die Acylierung der Hydroxylgruppen nach bekannten Verfahren (Fettsäurechlorid, z. B. Palmitsäurechlorid oder auch freier Fettsäure).

Die Zielprodukte werden dann durch PD/C katalysierte Hydrogenolyse gefolgt von Methylabspaltung mit Lithiumbromid erhalten.

Beispielhaft sollen einige Zielprodukte in Formelbildern, z. B. als Dipalmitoylester dargestellt werden:

### Phosphatidyldiglycerine und Analoga

Phosphatidyl-diglycerine und Phosphatidyl-triglycerine sind aus Phosphatidylglycerin, einem natürlicherweise vorkommenden Membranphospholipid entwickelt worden. überraschenderweise führt die Einführung eines weiteren Glycerinrests, der über eine Etherbrücke mit Phosphatidylglycerin verknüpft ist, zu besonderen Eigenschaften: Die Zirkulationszeiten von Liposomen im Blut, die dieses Phospholipid beinhalten, werden verändert und enorm verlängert. 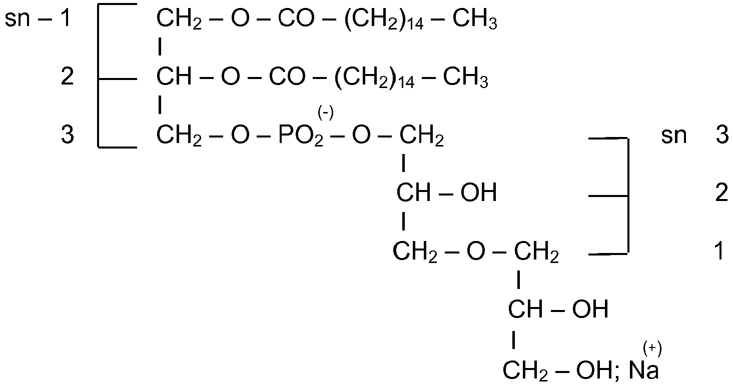 (sn)-1,2-Dipalmitoyl-glycero-3-phospho-sn-1-glycero-glycerin, Natriumsalz 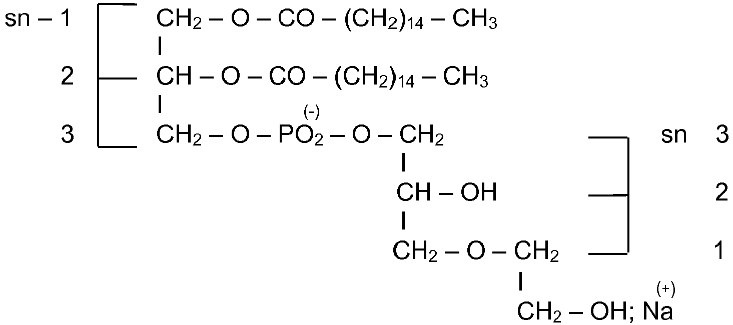 (sn)-1.2-Dipalmitoyl-sn-glycero-3-phospho-glyceroglykol, Natriumsalz

### Kardiolipine und Strukturanaloga

Kardiolipine und Analoga, die ebenfalls in natürlichen Membranen vorkommen, haben ebenfalls Zirkulationszeit verändernde Eigenschaften und erhöhen die Zirkulationszeit von Liposomen im Blut.

Es können mit den von uns entwickelten Bausteinen auch gemischtkettige Kardiolipine, also z.B. auch Strukturen mit nur drei Fettsäureresten hergestellt werden. 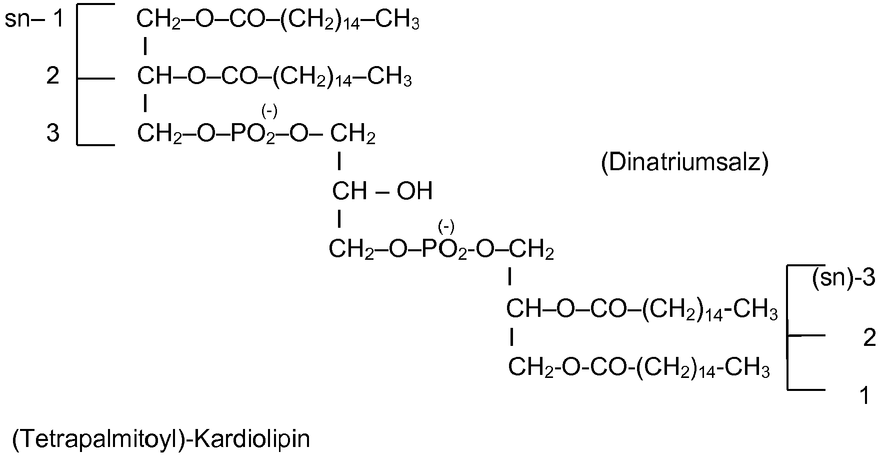 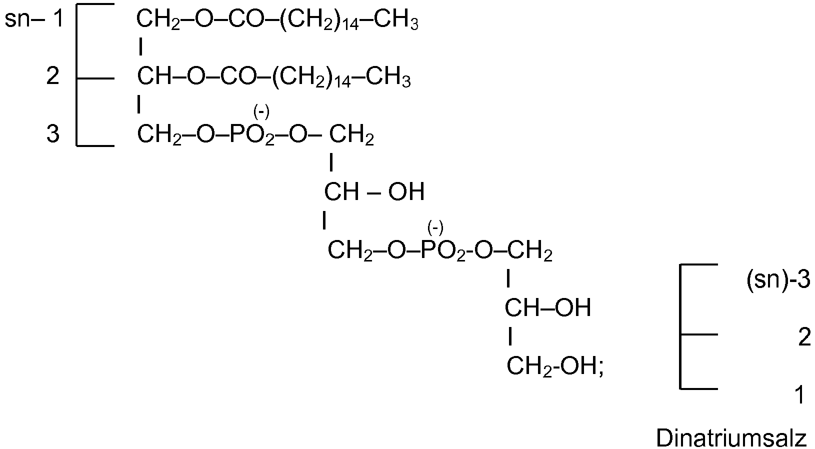 (sn)-1.2-Dipalmitoyl-3-glycero-phospho-glycero-(sn)-3-phosphoglycerin, Dinatriumsalz

### 7. Beispiele für (sn)-1.2-Diacyl-3-glycero-phosphol-(sn)-1-diglycerin

**1) Dipalmitoyl-Verbindung**
   Natriumsalz; C₄₁ H₈₀ Na O₁₂ P (MG 819.04)
**2) Dimyristoyl-Verbindung**
   Natriumsalz; C₃₇ H₇₂ Na O₁₂ P (MG 762.93)
**3) Distearoyl-Verbindung**
   Natriumsalz; C₄₅ H₈₈ Na O₁₂ P (MG 875.14)
**4) 1-Palmitoyl-2-Lauroyl-Verbindung**
   Natriumsalz; C₃₇ H₇₂ Na O₁₂ P (MG 762.93)
**5) 1-Stearoyl-2-Lauroyl-Verbindung**
   Natriumsalz; C₃₉ H₇₆ Na O₁₂ P (MG 790.98)
**6) 1-Stearoyl-2-Myristoyl-Verbindung**
   Natriumsalz; C₄₁ H₈₀ Na O₁₂ P (MG 819.04)
**7) 1-Stearoyl-2-Palmitoyl-Verbindung**
   Natriumsalz; C₄₃ H₈₄ Na O₁₂ P (MG 847.09)

### 8. Beispiele für (sn)-1.2-Diacyl-3-glycero-phospho-(sn)-1-glycero-glykol

**1) Dipalmitoyl-Verbindung**
   Natriumsalz; C₄₀ H₈₀ Na O₁₂ P (MG 789.04)
**2) Distearoyl-Verbindung**
   Natriumsalz; C₄₄ H₈₆ Na O₁₂ P (MG 845.14)
**3) 1-Stearoyl-2-Myristoyl-Verbindung**
   Natriumsalz; C₄₀ H₇₈ Na O₁₂ P (MG 789.04)
**4) 1-Stearoyl-2-Palmitoyl-Verbindung**
   Natriumsalz; C₄₂ H₈₂ Na O₁₂ P (MG 817.09)

### 9. Beispiele für (sn)-1.2-Diacyl-3-glycero-phospho-(sn)-1-diglycero-glykol

**1) Dipalmitoyl-Verbindung**
   Natriumsalz; C₄₃ H₈₄ Na O₁₂ P (MG 863.12)
**2) Distearoyl-Verbindung**
   Natriumsalz; C₄₇ H₉₀ Na O₁₂ P (MG 919.22)

### 10. Beispiele für (sn)-1.2-Diacyl-3-glycero-phospho-(sn)-1-triglycerin

**1) Dipalmitoyl-Verbindung**
   Natriumsalz; C₄₄ H₈₆ Na _{O12} P (MG 893.12)
**2) Distearoyl-Verbindung**
   Natriumsalz; C₄₈ H₉₂ Na O₁₂ P (MG 949.22)

Hierin werden auch die folgenden Items beschrieben:
Item 1 Stimuli-sensitives Nanocarriersystem, insbesondere thermosensitives Liposom,
dadurch gekennzeichnet,
dass es mindestens ein Phosphatidyloligoglycerin der Formel (IIa) 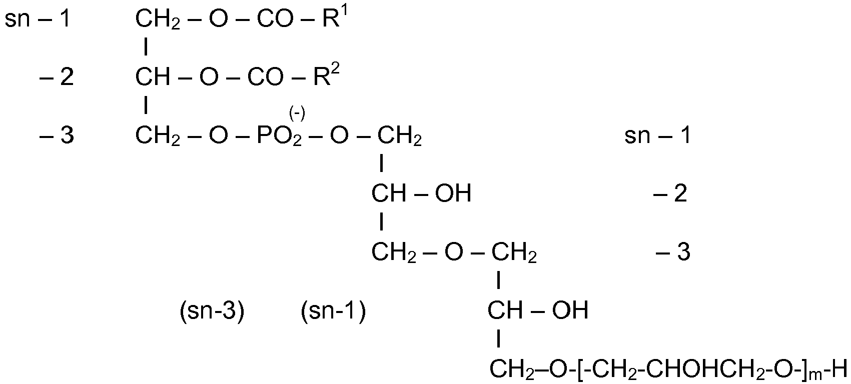 umfasst, worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.
Item 2 Stimuli-sensitives Nanocarriersystem gemäß Item 1,
dadurch gekennzeichnet,
dass es sich um ein thermosensitives Liposom handelt.
Item 3 Stimuli-sensitives Nanocarriersystem, insbesondere thermosensitives Liposom, gemäß einem der Items 1 oder 2,
dadurch gekennzeichnet,
dass es
(I) mindestens ein Phosphatidylcholin mit einer Hauptumwandlungstemperatur von 0 °C bis 80 °C und
(ii) mindestens ein Phosphatidyloligoglycerin umfasst.

Item 4 Stimuli-sensitives Nanocarriersystem, insbesondere thermosensitives Liposom, nach einem der Items 1 bis 3,
dadurch gekennzeichnet,
dass R¹ und R² unabhängig voneinander ein linearer gesättigter C12- bis C24-Alkylrest sind und m 0 oder 1 ist.
Item 5 Stimuli-sensitives Nanocarriersystem, insbesondere thermosensitives Liposom, nach einem der Items 1 bis 4,
dadurch gekennzeichnet, dass es mindestens ein Phosphatidyldiglycerin oder/und mindestens ein Phosphatidyltriglycerin umfasst.
Item 6 Stimuli-sensitives Nanocarriersystem, insbesondere thermosensitives Liposom, nach einem der Items 1 bis 5, umfassend ein Phosphatidylcholin der Formel (I) 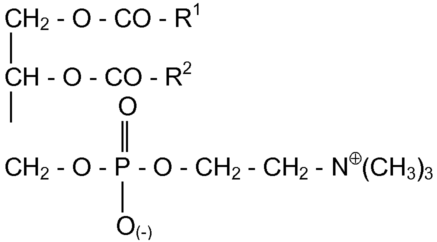 worin R¹ und R² jeweils unabhängig einen Kohlenwasserstoffrest mit 12 bis 24 C-Atomen darstellen.
Item 7 Stimuli-sensitives Nanocarriersystem, insbesondere thermosensitives Liposom, nach einem der vorhergehenden Items,
**dadurch gekennzeichnet,**
dass es mindestens ein Phosphatidylcholin enthält, ausgewählt aus der Gruppe bestehend aus 1-Palmitoyl-2-oleoylglycero-3-phosphocholin, 1-Stearoyl-2-oleoyl-3-phosphocholin, 1-Palmitoyl-2-lauroylglycero-3-phosphocholin, 1-Behenoyl-2-oleoylglycero-3-phosphocholin, 1-Stearoyl-2-lauroylglycero-3-phosphocholin, 1,3-Dimyristoylglycero-2-phosphocholin, 1,2-Dimyristoylglycero-3-phosphocholin, 1-Palmitoyl-2-myristoylglycero-3-phosphocholin, 1-Stearoyl-2-myristoylglycero-3-phosphocholin, 1-Myristoyl-2-palmitoylglycero-3-phosphocholin, 1,3-Palmitoylglycero-2-phosphocholin, 1,2-Dipalmitoylglycero-3-phosphocholin, 1-Myristoyl-2-stearoylglycero-3-phosphocholin, 1-Stearoyl-3-myristoylglycero-2-phosphocholin, 1-Stearoyl-2-palmitoylglycero-3-phosphocholin, 1-Palmitoyl-2-stearoylglycero-3-phosphocholin, 1,3-Distearoylglycero-2-phosphocholin, 1,2-Distearoylglycero-3-phosphocholin, 1,2-Diarachinoylglycero-3-phosphocholin, 1,2-Dibehenoylglycero-3-phosphocholin und 1,2-Dilignoceroylglycero-3-phosphocholin.
Item 8 Stimuli-sensitives Nanocarriersystem, insbesondere thermosensitives Liposom, nach einem der vorhergehenden Items,
**dadurch gekennzeichnet,**
dass es mindestens ein Phosphatidylcholin mit einer Hauptumwandlungstemperatur im Bereich von 35 °C bis 42 °C oder im Bereich von 40 °C bis 43 °C umfasst.
Item 9 Stimuli-sensitives Nanocarriersystem, insbesondere thermosensitives Liposom, nach Item 7 oder 8,
**dadurch gekennzeichnet,**
dass es mindestens ein Phosphatidylcholin, ausgewählt aus 1,3-Dipalmitoylphosphatidylcholin und 1,2-Dipalmitoylphosphatidylcholin, umfasst.
Item 10 Stimuli-sensitives Nanocarriersystem, insbesondere thermosensitives Liposom, nach einem der vorhergehenden Items,
**dadurch gekennzeichnet,**
dass es kein Cholesterin enthält.
Item 11 Stimuli-sensitives Nanocarriersystem, insbesondere thermosensitives Liposom, nach einem der vorhergehenden Items zur Verwendung zur Behandlung von Tumoren.
Item 12 Stimuli-sensitives Nanocarriersystem, insbesondere thermosensitives Liposom, nach Item 11 zur Verwendung zur Behandlung von Weichteilsarkom, Osteosarkom, Blasenkarzinom (muskelinvasiv, *muscle invasive bladder cancer* [MIBC] und nichtmuskelinvasiv, *non-muscle invasive bladder cancer* [NMIBC]), Ovarialkarzinom, Magenkarzinom, Mammakarzinom (v.a. triple negatives Mamma-Ca, [TNBC]), Hepatozelluläres Karzinom, Uteruskarzinom, Schilddrüsenkarzinom, Kopf-Hals-Tumoren, Prostatakarzinom, Chordom, Desmoidtumor, Glioblastom und andere Tumorerkrankungen mit vorzugsweise lokoregionärer Ausbreitung.
Item 13 Stimuli-sensitives Nanocarriersystem, insbesondere thermosensitives Liposom, nach Item 11 oder 12 zur Verwendung zur Behandlung von Harnblasentumoren.
Item 14 Stimuli-sensitives Nanocarriersystem, insbesondere thermosensitives Liposom, gemäß einem der Items 11 bis 13, weiterhin umfassend einen Wirkstoff, insbesondere ausgewählt aus Anthrazykline (z.B. Doxorubicin, Epirubicin), Oxazaphosphorine (z.B. Hydroxyifosfamid), Platinanaloga (Cisplatin, Oxaliplatin, Carboplatin), Gemcitabin, 5-Fluoruracil, Paclitaxel, Docetaxel, Etoposid, Topotecan, Vincristin, Irinotecan, Methotrexat, Bleomycin, Tyrosinkinaseinhibitoren, small molecules, DNA-Therapeutika, Radiosensitizer (in Verbindung mit Strahlentherapie).
Item 15 Stimuli-sensitives Nanocarriersystem, insbesondere thermosensitives Liposom, gemäß einem der Items 1 bis 10 zur Verwendung zur Behandlung von Infektionserkrankungen.
Item 16 Stimuli-sensitives Nanocarriersystem, insbesondere thermosensitives Liposom, gemäß Item 15,
dadurch gekennzeichnet,
dass die Infektionserkrankung durch Bakterien, Viren, Pilze oder/und Parasiten hervorgerufen ist.
Item 17 Stimuli-sensitives Nanocarriersystem, insbesondere thermosensitives Liposom, gemäß Item 15 oder 16 zur Behandlung von Infektionen von medizinischen Implantaten, insbesondere orthopädischer Prothesen, zur Behandlung von lokalisierten Infektionen, insbesondere Infektionen der tiefen Weichgewebe oder/und des Knochens, und/oder zur Therapie multiresistenter Erreger.
Item 18 Stimuli-sensitives Nanocarriersystem, insbesondere thermosensitives Liposom, gemäß einem der Items 15 bis 17, weiterhin umfassend einen Wirkstoff, insbesondere ausgewählt aus Antibiotika, Virostatika, Fungiziden und antiparasitär wirkenden Arzneistoffen.
Item 19 Stimuli-sensitives Nanocarriersystem, insbesondere thermosensitives Liposom, gemäß einem der Items 15 bis 18, dadurch gekennzeichnet,
dass der Wirkstoff ausgewählt ist aus
Antibiotika, insbesondere β-Lactame, Glykopeptide, Polyketide, Aminoglycosid-Antibiotika, Polypeptid-Antibiotika, Chinolone, Sulfonamide (z.B. Linezolid, Flucloxacillin, Cefazolin, Clindamycin, Vancomycin, Teicoplanin, Rifampicin Ampicillin, Ceftazidim, Ceftriaxon, Cefepim, Piperacillin, Fluorchinolone, Metronidazol, Amikacin, etc.) oder/und
Virostatika, insbesondere Entry-Inhibitoren, Penetrations-Inhibitoren, DNA-Polymerase-Inhibitoren, DNA/RNA-Polymerase-Inhibitoren, Reverse Transkriptase-Inhibitoren, Inosinmonophosphat-Dehydrogenase-Hemmer, Proteaseinhibitoren, Integrase-Inhibitoren, Helikase-Primase-Inhibitoren, Cyclophilin-Inhibitoren, Maturations-Inhibitoren, Terminase-Inhibitoren, Neuraminidase-Inhibitoren, etc. oder/und Fungiziden, insbesondere Azole (Benzimidazole ("MBC"), Triazole, Imidazole), Morpholine, Strobilurine, Chinoline, Anilino-Pyrimidine, Oxazolidin-dione, Carbonsäureamide, etc.
Item 20 Stimuli-sensitives Nanocarriersystem, insbesondere thermosensitives Liposom, gemäß einem der Items 1 bis 10 zur Verwendung zur Behandlung von Erkrankungen des Auges.
Item 21 Stimuli-sensitives Nanocarriersystem, insbesondere thermosensitives Liposom, gemäß Item 20 zur Behandlung von entzündlichen, degenerativen, infektiösen und/oder neoplastischen Erkrankungen des Auges, Wundheilungsstörungen oder/und Glaukom.
Item 22 Stimuli-sensitives Nanocarriersystem, insbesondere thermosensitives Liposom, gemäß einem der Items 1 bis 10 zur Verwendung zur Behandlung von Autoimmunerkrankungen.
Item 23 Stimuli-sensitives Nanocarriersystem, insbesondere thermosensitives Liposom, gemäß Item 22 zur Verwendung zur Behandlung von rheumatoider Arthritis oder/und chronisch entzündlichen Darmerkrankungen.
Item 24 Stimuli-sensitives Nanocarriersystem, insbesondere thermosensitives Liposom, gemäß Item 22 oder 23, weiterhin umfassend einen Wirkstoff, insbesondere ein Steroid, TNF-α oder/und Immunsuppressiva.
Item 25 Stimuli-sensitives Nanocarriersystem, insbesondere thermosensitives Liposom, gemäß einem der Items 1 bis 10 zur Verwendung zur Diagnose.
Item 26 Stimuli-sensitives Nanocarriersystem, insbesondere thermosensitives Liposom, gemäß Item 25 zur nicht-invasiven Temperaturmessung mit MR-Kontrastmittel und MR-Bildgebung.
Item 27 Stimuli-sensitives Nanocarriersystem, insbesondere thermosensitives Liposom, gemäß Item 25 oder 26, weiterhin umfassend einen Wirkstoff, insbesondere ein CT oder MRT-Kontrastmittel, vorzugsweise ausgewählt aus jodhaltigen Kontrastmitteln oder Gadolinium-Chelaten.
Item 28 Stimuli-sensitives Nanocarriersystem, insbesondere thermosensitives Liposom, gemäß einem der Items 1 bis 10 zur Verwendung zur Behandlung von degenerativen Erkrankungen.
Item 29 Stimuli-sensitives Nanocarriersystem, insbesondere thermosensitives Liposom, gemäß Item 28 zur lokalisierten Freisetzung von entzündungshemmenden Mitteln, Schmerzmitteln oder/und Chondroprotektiva.
Item 30 Stimuli-sensitives Nanocarriersystem, insbesondere thermosensitives Liposom, gemäß einem der Items 1 bis 10 zur Verwendung zur Behandlung von dementiellen Syndromen, Morbus Alzheimer oder/und fokalen neurologisch psychiatrischen Erkrankungen, insbesondere Epilepsie.
Item 31 Stimuli-sensitives Nanocarriersystem, insbesondere thermosensitives Liposom, gemäß einem der Items 1 bis 10 zur Verwendung zur Behandlung von Atherosklerose.
Item 32 Stimuli-sensitives Nanocarriersystem, insbesondere thermosensitives Liposom, gemäß einem der Items 1 bis 10 zur Verwendung zur Behandlung von Thrombosen.
Item 33 Stimuli-sensitives Nanocarriersystem, insbesondere thermosensitives Liposom, gemäß Item 32, weiterhin umfassend wenigstens einen Wirkstoff, insbesondere ausgewählt aus Fibrinolytika, vorzugsweise Streptokinase, Urokinase oder/und Alteplase.
Item 34 Stimuli-sensitives Nanocarriersystem nach einem der vorhergehenden Items, wobei das stimuli-sensitives Nanocarriersystem durch Ausübung eines Stimulus verändert und insbesondere geöffnet wird, um einen gegebenenfalls enthaltenen Wirkstoff aus dem Nanocarriersystem freizusetzen.
Item 35 Stimuli-sensitives Nanocarriersystem gemäß Item 34, wobei der Stimulus ausgewählt wird aus Radiofrequenz (z.B. radiative Oberflächen- und Tiefenhyperthermiesysteme, Blasenhyperthermiesysteme), Ultraschall (z.B. hochfokussierter Ultraschall [*high intensity focused ultrasound*, HIFU], niedrigintensiver Ultraschall [*low intensity focused ultrasound*, LIFU]), Licht, Laser, Konduktion durch erwärmte Flüssigkeit, andere physikalische Prinzipien, die entweder zu einer lokoregionären Erwärmung führen und/oder Membranen bestehend aus Phospholipiden destabilisieren können.
Item 36 Stimuli-sensitives Nanocarriersystem, insbesondere thermosensitives Liposom, nach einem der vorhergehenden Items 11 bis 13, **dadurch gekennzeichnet,** dass es weiterhin ein Zytostatikum umfasst.
Item 37 Stimuli-sensitives Nanocarriersystem, insbesondere thermosensitives Liposom, nach Item 36, **dadurch gekennzeichnet,** dass das Zytostatikum ausgewählt ist aus der Gruppe bestehend aus Mitomycin C, Doxorubicin, Epirubicin, Gemcitabin, Trabectedin, Cisplatin, Carboplatin und Oxoliplatin.
Item 38 Stimuli-sensitives Nanocarriersystem, insbesondere thermosensitives Liposom, nach einem der vorhergehenden Items in Kombination mit Hyperthermie oder/und Ultraschall.
Item 39 Stereospezifisches Phosphatidyloligoglycerin der Formel (IIa)

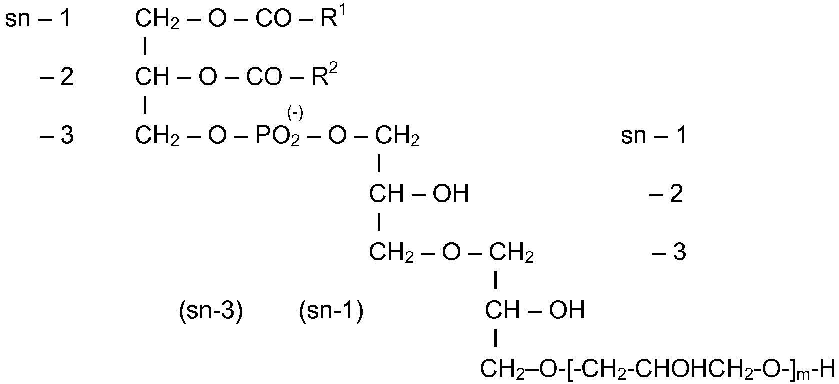

| | |
|---|---|
| Konfiguration : | natürlich |
| Bezeichnung : | 1.2-Diacyl-sn-3-glycero-phospho-sn-1-oligoglycerin; |
| | sn, stereospezifische Nummerierung; |

worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.
Item 40 Stimuli-sensitives Liposom, umfassend ein sterespezifisches Phosphatidyloligoglycerin der Formel (IIa) gemäß Item 39 zur Verwendung zur Behandlung von Harnblasentumoren.
Item 41 Stimuli-sensitives Liposom gemäß Item 40, weiterhin umfassend ein Phosphatidylcholin der Formel (I) mit einer Hauptumwandlungstemperatur von 35 bis 45 °C, insbesondere 40 bis 43 °C.

## Patentansprüche

1. Stimuli-sensitives Nanocarriersystem zur Verwendung zur lokoregionären Therapie,
**dadurch gekennzeichnet,**
**dass** es mindestens ein Phosphatidyloligoglycerin der Formel (IIa) 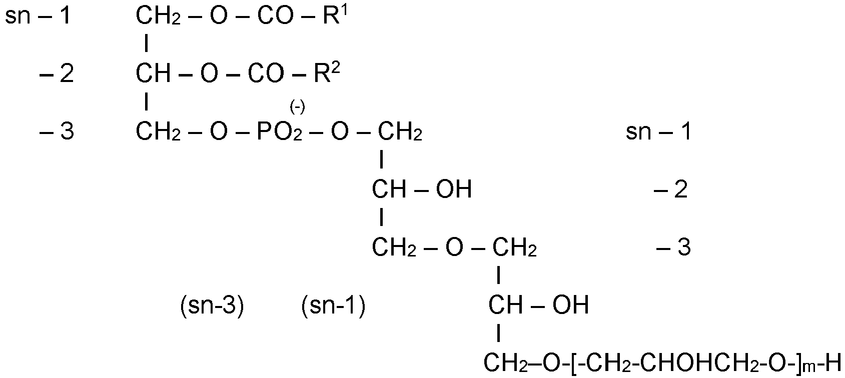 umfasst, worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.

2. Stimuli-sensitives Nanocarriersystem zur Verwendung nach Anspruch 1,
**dadurch gekennzeichnet,**
**dass** es sich um ein thermosensitives Liposom handelt.

3. Stimuli-sensitives Nanocarriersystem, insbesondere thermosensitives Liposom, zur Verwendung nach Anspruch 1 oder 2
**dadurch gekennzeichnet,**
**dass** es
(i) mindestens ein Phosphatidylcholin mit einer Hauptumwandlungstemperatur von 0 °C bis 80 °C und
(ii) mindestens ein Phosphatidyloligoglycerin umfasst und/oder
**dadurch gekennzeichnet,**
**dass** R¹ und R² unabhängig voneinander ein linearer gesättigter C12- bis C24-Alkylrest sind und m 0 oder 1 ist und/oder
**dadurch gekennzeichnet,**
**dass** es mindestens ein Phosphatidyldiglycerin oder/und mindestens ein Phosphatidyltriglycerin umfasst.

4. Stimuli-sensitives Nanocarriersystem, insbesondere thermosensitives Liposom, zur Verwendung nach einem der Ansprüche 1 bis 3, umfassend ein Phosphatidylcholin der Formel (I) 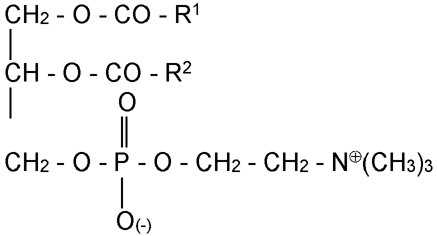 worin R¹ und R² jeweils unabhängig einen Kohlenwasserstoffrest mit 12 bis 24 C-Atomen darstellen und/oder
**dadurch gekennzeichnet,**
**dass** es mindestens ein Phosphatidylcholin enthält, ausgewählt aus der Gruppe bestehend aus 1-Palmitoyl-2-oleoylglycero-3-phosphocholin, 1-Stearoyl-2-oleoyl-3-phosphocholin, 1-Palmitoyl- 2-lauroylglycero-3-phosphocholin, 1-Behenoyl-2-oleoylglycero-3-phosphocholin, 1-Stearoyl-2-lauroylglycero-3-phosphocholin, 1,3-Dimyristoylglycero-2-phosphocholin, 1,2-Dimyristoylglycero-3-phosphocholin, 1-Palmitoyl-2-myristoylglycero-3-phosphocholin, 1-Stearoyl-2-myristoylglycero-3-phosphocholin, 1-Myristoyl-2-palmitoylglycero-3-phosphocholin, 1,3-Palmitoylglycero-2-phosphocholin, 1,2-Dipalmitoylglycero-3-phosphocholin, 1-Myristoyl-2-stearoylglycero-3-phosphocholin, 1-Stearoyl-3-myristoylglycero-2-phosphocholin, 1-Stearoyl-2-palmitoylglycero-3-phosphocholin, 1-Palmitoyl-2-stearoylglycero-3-phosphocholin, 1,3-Distearoylglycero-2-phosphocholin, 1,2-Distearoylglycero-3-phosphocholin, 1,2-Diarachinoylglycero-3-phosphocholin, 1,2-Dibehenoylglycero-3-phosphocholin und 1,2-Dilignoceroylglycero-3-phosphocholin und/oder
**dadurch gekennzeichnet,**
**dass** es mindestens ein Phosphatidylcholin mit einer Hauptumwandlungstemperatur im Bereich von 35 °C bis 42 °C oder im Bereich von 40 °C bis 43 °C umfasst und/oder
**dadurch gekennzeichnet,**
**dass** es mindestens ein Phosphatidylcholin, ausgewählt aus 1,3-Dipalmitoylphosphatidylcholin und 1,2-Dipalmitoylphosphatidylcholin, umfasst.

5. Stimuli-sensitives Nanocarriersystem, insbesondere thermosensitives Liposom, zur Verwendung nach einem der vorhergehenden Ansprüche,
**dadurch gekennzeichnet,**
**dass** es kein Cholesterin enthält.

6. Stimuli-sensitives Nanocarriersystem, insbesondere thermosensitives Liposom, nach einem der vorhergehenden Ansprüche zur Verwendung zur Behandlung von Tumoren, insbesondere zur Verwendung zur Behandlung von Weichteilsarkom, Osteosarkom, Blasenkarzinom, Ovarialkarzinom, Magenkarzinom, Mammakarzinom, Hepatozelluläres Karzinom, Uteruskarzinom, Schilddrüsenkarzinom, Kopf-Hals-Tumoren, Prostatakarzinom, Chordom, Desmoidtumor, Glioblastom oder/und anderen Tumorerkrankungen mit vorzugsweise lokoregionärer Ausbreitung und bevorzugt zur Verwendung zur Behandlung von Harnblasentumoren.

7. Stimuli-sensitives Nanocarriersystem, insbesondere thermosensitives Liposom, zur Verwendung nach Anspruch 6, weiterhin umfassend einen Wirkstoff, insbesondere ausgewählt aus Anthrazyklinen, Oxazaphosphorinen, Platinanaloga, Gemcitabin, 5-Fluoruracil, Paclitaxel, Docetaxel, Etoposid, Topotecan, Vincristin, Irinotecan, Methotrexat, Bleomycin, Tyrosinkinaseinhibitoren, small molecules, DNA-Therapeutika und Radiosensitizer oder
weiterhin umfassend ein Zytostatikum, insbesondere ausgewählt aus der Gruppe bestehend aus Mitomycin C, Doxorubicin, Epirubicin, Gemcitabin, Trabectedin, Cisplatin, Carboplatin und Oxoliplatin.

8. Stimuli-sensitives Nanocarriersystem, insbesondere thermosensitives Liposom, nach einem der Ansprüche 1 bis 5 zur Verwendung zur Behandlung von Infektionserkrankungen.

9. Stimuli-sensitives Nanocarriersystem, insbesondere thermosensitives Liposom, zur Verwendung nach Anspruch 8,
**dadurch gekennzeichnet,**
**dass** die Infektionserkrankung durch Bakterien, Viren, Pilze oder/und Parasiten hervorgerufen ist und/oder zur Behandlung von Infektionen von medizinischen Implantaten, zur Behandlung von lokalisierten Infektionen und/oder zur Therapie multiresistenter Erreger und/oder
weiterhin umfassend einen Wirkstoff, insbesondere ausgewählt aus Antibiotika, Virostatika, Fungiziden und antiparasitär wirkenden Arzneistoffen,
wobei der Wirkstoff insbesondere ausgewählt ist aus Antibiotika, insbesondere β-Lactame, Glykopeptide, Polyketide, Aminoglycosid-Antibiotika, Polypeptid-Antibiotika, Chinolone, Sulfonamide oder/und
Virostatika, insbesondere Entry-Inhibitoren, Penetrations-Inhibitoren, DNA-Polymerase-Inhibitoren, DNA/RNA-Polymerase-Inhibitoren, Reverse Transkriptase-Inhibitoren, Inosinmonophosphat-Dehydrogenase-Hemmer, Proteaseinhibitoren, Integrase-Inhibitoren, Helikase-Primase-Inhibitoren, Cyclophilin-Inhibitoren, Maturations-Inhibitoren, Terminase-Inhibitoren, Neuraminidase-Inhibitoren, etc. oder/und Fungiziden, insbesondere Azole, Morpholine, Strobilurine, Chinoline, Anilino-Pyrimidine, Oxazolidin-dione, Carbonsäureamide.

10. Stimuli-sensitives Nanocarriersystem, insbesondere thermosensitives Liposom, zur Verwendung nach einem der Ansprüche 1 bis 5 zur Verwendung zur Behandlung von Erkrankungen des Auges,
insbesondere zur Behandlung von entzündlichen, degenerativen, infektiösen und/oder neoplastischen Erkrankungen des Auges, Wundheilungsstörungen oder/und Glaukom oder
zur Verwendung zur Behandlung von Autoimmunerkrankungen, insbesondere zur Verwendung zur Behandlung von rheumatoider Arthritis oder/und chronisch entzündlichen Darmerkrankungen und/oder
insbesondere weiterhin umfassend einen Wirkstoff, insbesondere ein Steroid, TNF-α oder/und Immunsuppressiva.

11. Stimuli-sensitives Nanocarriersystem, insbesondere thermosensitives Liposom, nach einem der Ansprüche 1 bis 10 zur Verwendung zur Diagnose,
insbesondere zur nicht-invasiven Temperaturmessung mit MR-Kontrastmittel und MR-Bildgebung und/oder
insbesondere weiterhin umfassend einen Wirkstoff, insbesondere ein CT oder MRT-Kontrastmittel, vorzugsweise ausgewählt aus jodhaltigen Kontrastmitteln oder Gadolinium-Chelaten.

12. Stimuli-sensitives Nanocarriersystem, insbesondere thermosensitives Liposom, nach einem der Ansprüche 1 bis 10 zur Verwendung zur Behandlung von degenerativen Erkrankungen,
insbesondere zur lokalisierten Freisetzung von entzündungshemmenden Mitteln, Schmerzmitteln oder/und Chondroprotektiva oder
zur Verwendung zur Behandlung von dementiellen Syndromen, Morbus Alzheimer oder/und fokalen neurologisch psychiatrischen Erkrankungen, insbesondere Epilepsie oder
zur Verwendung zur Behandlung von Atherosklerose.

13. Stimuli-sensitives Nanocarriersystem, insbesondere thermosensitives Liposom, nach einem der Ansprüche 1 bis 10 zur Verwendung zur Behandlung von Thrombosen insbesondere weiterhin umfassend wenigstens einen Wirkstoff, insbesondere ausgewählt aus Fibrinolytika, vorzugsweise Streptokinase, Urokinase oder/und Alteplase.

14. Stimuli-sensitives Nanocarriersystem, insbesondere thermosensitives Liposom, zur Verwendung nach einem der vorhergehenden Ansprüche in Kombination mit Hyperthermie oder/und Ultraschall.

15. Stereospezifisches Phosphatidyloligoglycerin der Formel (IIa) 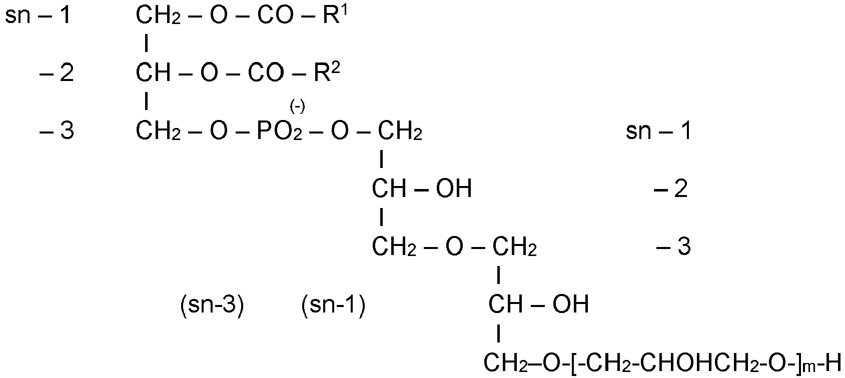 worin R¹ und R² jeweils unabhängig einen Kohlenwasserstoffrest mit 12 bis 24 C-Atomen darstellen und m eine ganze Zahl von 0 bis 50 ist, worin die Verknüpfung vom Glycerid zur Phosphatgruppe stereospezifisch ist und als sn-3-Verknüpfung vorliegt und die Verknüpfung von der Phosphatgruppe zum Oligoglycerin stereospezifisch ist und als sn-1-Verknüpfung vorliegt.

## Claims

1. Stimuli-sensitive nanocarrier system for use in locoregional therapy,
**characterised in that**
said system comprises at least one phosphatidyloligoglycerol of formula (Ila) 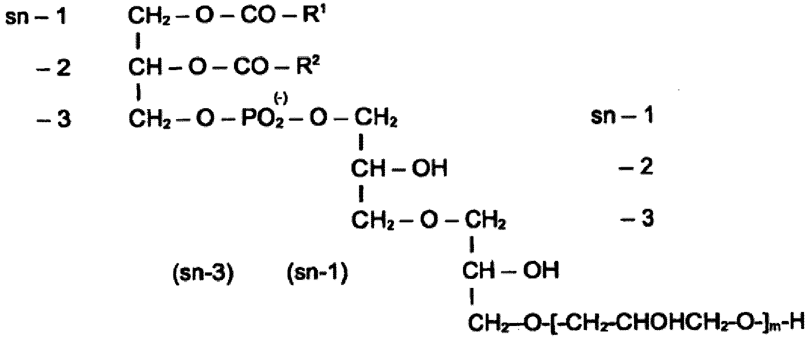 wherein R¹ and R² each independently represent a hydrocarbon functional group having from 12 to 24 carbon atoms and m is an integer from 0 to 50, wherein the linkage from the glyceride to the phosphate group is stereospecific and is in the form of an sn-3 linkage and the linkage from the phosphate group to the oligoglycerol is stereospecific and is in the form of an sn-1 linkage.

2. Stimuli-sensitive nanocarrier system for use according to claim 1,
**characterised in that**
said system is a thermosensitive liposome.

3. Stimuli-sensitive nanocarrier system, in particular thermosensitive liposome, for use according to either claim 1 or claim 2,
**characterised in that**
said system comprises
(i) at least one phosphatidylcholine having a main transition temperature of from 0 °C to 80 °C and,
(ii) at least one phosphatidyloligoglycerol and/or
**characterised in that**
R¹ and R² independently of one another are a linear saturated C12- to C24-alkyl functional group and m is 0 or 1 and/or
**characterised in that**
said system comprises at least one phosphatidyldiglycerol and/or at least one phosphatidyltriglycerol.

4. Stimuli-sensitive nanocarrier system, in particular thermosensitive liposome, for use according to any of claims 1 to 3, comprising a phosphatidylcholine of formula (I) 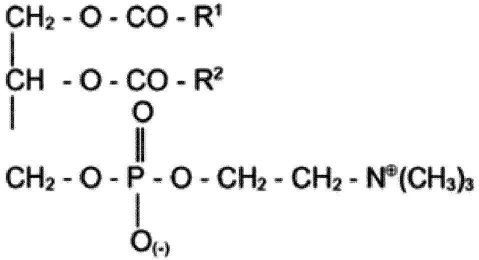 wherein R¹ and R² each independently represent a hydrocarbon functional group having from 12 to 24 carbon atoms and/or
**characterised in that**
said system comprises at least one phosphatidylcholine selected from the group consisting of 1-palmitoyl-2-oleoylglycero-3-phosphocholine, 1-stearoyl-2-oleoyl-3-phosphocholine, 1-palmitoyl-2-lauroylglycero-3-phosphocholine, 1-behenoyl-2-oleoylglycero-3-phosphocholine, 1-stearoyl-2-lauroylglycero-3-phosphocholine, 1,3-dimyristoylglycero-2-phosphocholine, 1,2-dimyristoylglycero-3-phosphocholine, 1-palmitoyl-2-myristoylglycero-3-phosphocholine, 1-stearoyl-2-myristoyl-glycero-3-phosphocholine, 1-myristoyl-2-palmitoylglycero-3-phosphocholine, 1,3-palmitoylglycero-2-phosphocholine, 1,2-dipalmitoylglycero-3-phosphocholine, 1-myristoyl-2 stearoylglycero-3-phosphocholine, 1-stearoyl-3 myristoylglycero-2-phosphocholine, 1-stearoyl-2-palmitoylglycero-3-phosphocholine, 1 palmitoyl-2-stearoylglycero-3-phosphocholine, 1,3-distearoylglycero-2-phosphocholine, 1,2-distearoylglycero-3-phosphocholine, 1,2-diarachinoylglycero-3-phosphocholine, 1,2-dibehenoylglycero-3-phosphocholine and 1,2-dilignoceroylglycero-3-phosphocholine and/or
**characterised in that**
said system comprises at least one phosphatidylcholine having a main transition temperature in the range of from 35 °C to 42 °C or in the range of from 40 °C to 43 °C and/or
**characterised in that**
said system comprises at least one phosphatidylcholine selected from 1,3-dipalmitoyl-phosphatidylcholine or 1,2-dipalmitoylphosphatidylcholine.

5. Stimuli-sensitive nanocarrier system, in particular thermosensitive liposome, for use according to any of the preceding claims,
**characterised in that**
said system does not comprise cholesterol.

6. Stimuli-sensitive nanocarrier system, in particular thermosensitive liposome, according to any of the preceding claims for use in the treatment of tumors,
in particular for use in the treatment of soft-tissue sarcoma, osteosarcoma, bladder carcinoma, ovarian carcinoma, stomach carcinoma, breast carcinoma, hepatocellular carcinoma, uterine carcinoma, carcinoma of the thyroid gland, head-neck tumor, prostate carcinoma, chordoma, desmoid tumour, glioblastoma and/or other tumor diseases having preferably locoregional spread and preferably for use in the treatment of bladder tumors.

7. Stimuli-sensitive nanocarrier system, in particular thermosensitive liposome, for use according to claim 6, further comprising an active ingredient, in particular selected from anthracyclines, oxazaphosphorines, platinum analogues, gemcitabine, 5-fluorouracil, paclitaxel, docetaxel, etoposide, topotecan, vincristine, irinotecan, methotrexate, bleomycin, tyrosine kinase inhibitors, small molecules, DNA therapeutics and radiosensitisers or
further comprising a cytostatic, in particular selected from the group consisting of mitomycin C, doxorubicin, epirubicin, gemcitabine, trabectedin, cisplatin, carboplatin and oxaliplatin.

8. Stimuli-sensitive nanocarrier system, in particular thermosensitive liposome, according to any of claims 1 to 5 for use in the treatment of infectious diseases.

9. A stimuli-sensitive nanocarrier system, in particular thermosensitive liposome, for use according to claim 8,
**characterised in that**
the infectious disease is caused by bacteria, viruses, fungi and/or parasites and/or for the treatment of infections of medical implants, for the treatment of localised infections and/or for the therapy of multiresistant pathogens and/or
further comprising an active ingredient, in particular selected from antibiotics, virostatics, fungicides and medicinal drugs having an anti-parasitic effect,
wherein the active ingredient is selected from antibiotics, in particular β-lactams, glycopeptides, polyketides, aminoglycoside antibiotics, polypeptide antibiotics, quinolones, sulfonamides, and/or
virostatics, in particular entry inhibitors, penetration inhibitors, DNA polymerase inhibitors, DNA/RNA polymerase inhibitors, reverse transcriptase inhibitors, inosine monophosphate dehydrogenase inhibitors, protease inhibitors, integrase inhibitors, helicase-primase inhibitors, cyclophilin inhibitors, maturation inhibitors, terminase inhibitors, neuraminidase inhibitors, etc., and/or fungicides, in particular azoles, morpholines, strobilurins, quinolines, anilino-pyrimidines, oxazolidine-diones, carboxylic acid amides.

10. Stimuli-sensitive nanocarrier system, in particular thermosensitive liposome, for use according to any of claims 1 to 5 for use in the treatment of diseases of the eye,
in particular for the treatment of inflammatory, degenerative, infectious and/or neoplastic diseases of the eye, wound healing disorders and/or glaucoma or
for use in the treatment of autoimmune diseases,
in particular for use in the treatment of rheumatoid arthritis and/or chronic inflammatory intestinal diseases and/or
in particular further comprising an active ingredient, in particular a steroid, TNF-α and/or immunosuppressants.

11. Stimuli-sensitive nanocarrier system, in particular thermosensitive liposome, according to any of claims 1 to 10 for use in diagnosis,
in particular for non-invasive temperature measurement using MR contrast agent and MR imaging and/or
in particular further comprising an active ingredient, in particular a CT or MRT contrast agent, preferably selected from iodine-containing contrast agents or gadolinium chelates.

12. Stimuli-sensitive nanocarrier system, in particular thermosensitive liposome, according to any of claims 1 to 10 for use in the treatment of degenerative diseases,
in particular for localised release of anti-inflammatory agents, analgesics and/or chondroprotectants or
for use in the treatment of dementia syndromes, Alzheimer's disease and/or a focal neurological psychiatric disease, in particular epilepsy or for use in the treatment of atherosclerosis.

13. Stimuli-sensitive nanocarrier system, in particular thermosensitive liposome, according to any of claims 1 to 10 for use in the treatment of thromboses,
in particular further comprising at least one active ingredient, in particular selected from fibrinolytics, preferably streptokinase, urokinase and/or alteplase.

14. Stimuli-sensitive nanocarrier system, in particular thermosensitive liposome, for use according to any of the preceding claims in combination with hyperthermia and/or ultrasound.

15. Stereospecific phosphatidyloligoglycerol of formula (Ila) 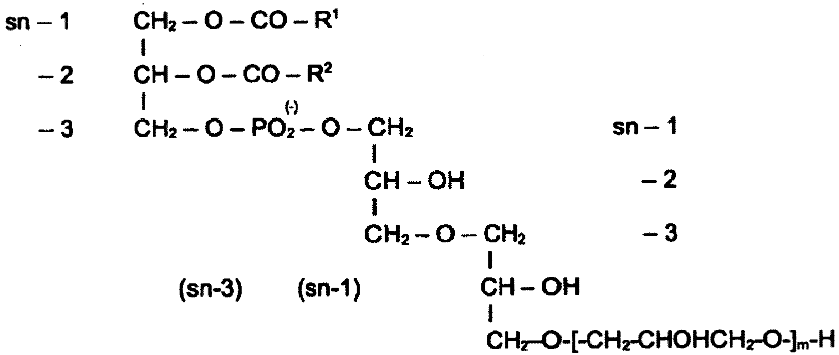 wherein R¹ and R² each independently represent a hydrocarbon functional group having from 12 to 24 carbon atoms and m is an integer from 0 to 50, wherein the linkage from the glyceride to the phosphate group is stereospecific and is in the form of an sn-3 linkage and the linkage from the phosphate group to the oligoglycerol is stereospecific and is in the form of an sn-1 linkage.

## Revendications

1. Système nanocarrier sensible aux stimuli pour l'utilisation dans la thérapie locorégionale,
**caractérisé en ce que**
il comprend au moins un phosphatidyloligoglycérol de la formule (IIa), 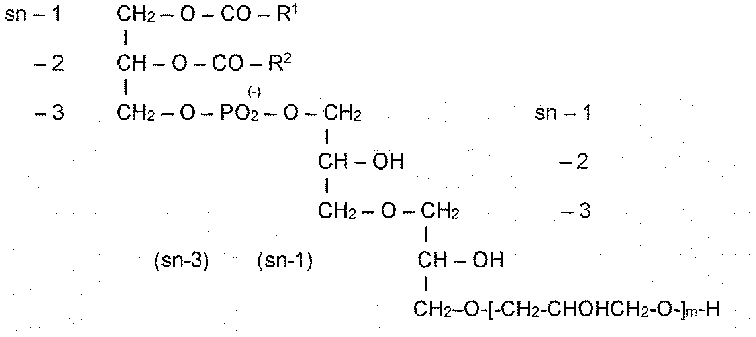 dans laquelle R¹ et R² représentent chacun indépendamment un radical hydrocarboné ayant de 12 à 24 atomes de carbone et m est un nombre entier de 0 à 50, dans laquelle la liaison du glycéride au groupe phosphate est stéréospécifique et est présente sous forme d'une liaison sn-3 et la liaison du groupe phosphate à l'oligoglycérol est stéréospécifique et est présente sous forme d'une liaison sn-1.

2. Système nanocarrier sensible aux stimuli pour l'utilisation selon la revendication 1,
**caractérisé en ce que**
il s'agit d'un liposome thermosensible.

3. Système nanocarrier sensible aux stimuli, en particulier un liposome thermosensible, pour l'utilisation selon la revendication 1 ou 2,
**caractérisé en ce que**
il comprend :
(i) au moins une phosphatidylcholine ayant une température de transformation principale de 0 °C à 80 °C, et
(ii) au moins un phosphatidyloligoglycérol et/ou
**caractérisé en ce que**
R¹ et R² sont indépendamment l'un de l'autre un radical alkyle linéaire saturé en C12 à C24 et m est 0 ou 1, et/ou
**caractérisé en ce que**
il comprend au moins un phosphatidyldiglycérol ou/et au moins un phosphatidyltriglycérol.

4. Système nanocarrier sensible aux stimuli, en particulier un liposome thermosensible, pour l'utilisation selon l'une quelconque des revendications 1 à 3, comprenant une phosphatidylcholine de la formule (I) 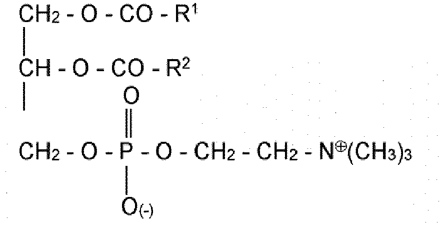 dans laquelle R¹ et R² représentent chacun indépendamment un radical hydrocarboné ayant de 12 à 24 atomes de carbone et/ou
**caractérisé en ce que**
il contient au moins une phosphatidylcholine choisie dans le groupe, comprenant 1-palmitoyl-2-oleoylglycéro-3-phosphocholine, 1-stéaroyl-2-oleoyl-3-phosphocholine, 1-palmitoyl-2-lauroylglycéro-3-phosphocholine, 1-behénoyl-2-oleoylglycéro-3-phosphocholine, 1-stéaroyl-2-lauroylglycéro-3-phosphocholine, 1,3-dimyristoylglycéro-2-phosphocholine, 1,2-dimyristoylglycéro-3-phosphocholine, 1-palmitoyl-2-myristoylglycéro-3-phosphocholine, 1-stéaroyl-2-myristoylglycéro-3-phosphocholine, 1-myristoyl-2-palmitoylglycéro-3-phosphocholine, 1,3-palmitoylglycéro-2-phosphocholine, 1,2-dipalmitoylglycéro-3-phosphocholine, 1-myristoyl-2-stearoylglycéro-3-phosphocholine, 1-stearoyl-3-myristoylglycéro-2-phosphocholine, 1-stearoyl-2-palmitoylglycero-3-phosphocholine, 1-palmitoyl-2-stearoylglycero-3-phosphocholine, 1,3-distéaroylglycéro-2-phosphocholine, 1,2-distéaroylglycéro-3-phosphocholine, 1,2-diarachinoylglycéro-3-phosphocholine, 1,2-diibehenoylglycéro-3-phosphocholine et 1,2-dilignoceroylglycéro-3-phosphocholine et/ou
**caractérisé en ce que**
il comprend au moins une phosphatidylcholine ayant une température de transformation principale comprise entre 35 °C et 42 °C ou entre 40 °C et 43 °C et/ou
**caractérisé en ce que**
il comprend au moins une phosphatidylcholine choisie parmi la 1,3-dipalmitoylphosphatidylcholine et la 1,2-dipalmitoylphosphatidylcholine.

5. Système nanocarrier sensible aux stimuli, en particulier un liposome thermosensible, pour l'utilisation selon l'une des revendications précédentes,
**caractérisé en ce que**
il ne comprend pas de cholestérol.

6. Système nanocarrier sensible aux stimuli, en particulier un liposome thermosensible, selon l'une des revendications précédentes pour l'utilisation dans le traitement des tumeurs, en particulier pour l'utilisation dans le traitement du sarcome des tissus mous, de l'ostéosarcome, du carcinome de la vessie, du carcinome des ovaires, du carcinome gastrique, du carcinome du sein, du carcinome hépatocellulaire, du carcinome utérin, du carcinome de la thyroïde, des tumeurs de la tête et du cou, du carcinome de la prostate, du chordome, du tumeur desmoïde, du glioblastome et/ou d'autres maladies tumorales à diffusion locorégionale de préférence et à utiliser de préférence dans le traitement des tumeurs de la vessie.

7. Système nanocarrier sensible aux stimuli, en particulier un liposome thermosensible, pour l'utilisation selon la revendication 6, comprenant en outre un agent actif, en particulier choisi parmi les anthracyclines, les oxazaphosphorines, les analogues du platine, la gemcitabine, le 5-fluorouracile, le paclitaxel, le docétaxel, l'étoposide, le topotécan, la vincristine, l'irinotécan, la méthotrexate, la bléomycine, les inhibiteurs de tyrosine kinase, les petites molécules, les produits thérapeutiques de l'ADN et les radiosensibilisateurs, ou
comprenant en outre un agent cytostatique, en particulier choisi dans le groupe, comprenant la mitomycine C, la doxorubicine, l'épirubicine, la gemcitabine, la trabectine, le cisplatine, le carboplatine et l'oxoliplatine.

8. Système nanocarrier sensible aux stimuli, en particulier un liposome thermosensible, selon l'une des revendications 1 à 5, pour l'utilisation dans le traitement des maladies infectieuses.

9. Système nanocarrier sensible aux stimuli, en particulier un liposome thermosensible, pour l'utilisation selon la revendication 8,
**caractérisé en ce que**
la maladie infectieuse est provoquée par des bactéries, des virus, des champignons et/ou des parasites et/ou
pour le traitement d'infections d'implants médicaux, pour le traitement d'infections localisées et/ou pour la thérapie de pathogènes multirésistants et/ou comprenant en outre une substance active, en particulier choisie parmi les antibiotiques, les antiviraux, les fongicides et les médicaments antiparasitaires, dans lequel le principe actif est notamment choisi parmi les antibiotiques, en particulier les β-lactames, les glycopeptides, les polycétides, les antibiotiques aminoglycosidiques, les antibiotiques polypeptidiques, les quinolones, les sulfonamides et/ou
les antiviraux, en particulier les inhibiteurs d'entrée, les inhibiteurs de pénétration, les inhibiteurs d'ADN polymérase, les inhibiteurs d'ADN/ARN polymérase, les inhibiteurs de transcriptase inverse, les inhibiteurs de l'inosine monophosphate déshydrogénase, les inhibiteurs de protéase, les inhibiteurs d'intégrase, les inhibiteurs d'hélicase-primase, les inhibiteurs de cyclophiline, les inhibiteurs de maturation, les inhibiteurs de terminase, les inhibiteurs de neuraminidase, etc. ou/et
des fongicides, notamment les azoles, les morpholines, les strobilurines, les quinoléines, les anilino-pyrimidines, les diones d'oxazolidine, les amides d'acides carboxyliques.

10. Système nanocarrier sensible aux stimuli, en particulier un liposome thermosensible, pour l'utilisation selon l'une quelconque des revendications 1 à 5 pour l'utilisation dans le traitement des maladies de l'oeil,
en particulier pour le traitement des maladies inflammatoires, dégénératives, infectieuses et/ou néoplasiques de l'oeil, des troubles de la cicatrisation et/ou du glaucome ou
pour l'utilisation dans le traitement des maladies auto-immunes, en particulier pour l'utilisation dans le traitement de la polyarthrite rhumatoïde et/ou des maladies inflammatoires de l'intestin et/ou
comprenant en outre un agent actif, en particulier un stéroïde, le TNF-α et/ou des immunosuppresseurs.

11. Système nanocarrier sensible aux stimuli, en particulier un liposome thermosensible, selon l'une quelconque des revendications 1 à 10, pour l'utilisation dans le diagnostic,
en particulier pour la mesure non invasive de la température avec un agent de contraste RM et l'imagerie RM et/ou
comprenant en outre un agent actif, en particulier un agent de contraste CT ou IRM, de préférence choisi parmi les agents de contraste contenant de l'iode ou les chélates de gadolinium.

12. Système nanocarrier sensible aux stimuli, en particulier un liposome thermosensible, selon l'une quelconque des revendications 1 à 10, pour l'utilisation dans le traitement des maladies dégénératives,
en particulier pour la libération localisée d'agents anti-inflammatoires, d'analgésiques et/ou de chondroprotecteurs, ou
pour l'utilisation dans le traitement des syndromes de démence, de la maladie d'Alzheimer et/ou des maladies neurologiques psychiatriques focales, en particulier l'épilepsie, ou pour l'utilisation dans le traitement de l'athérosclérose.

13. Système nanocarrier sensible aux stimuli, en particulier un liposome thermosensible, selon l'une quelconque des revendications 1 à 10, pour l'utilisation dans le traitement de la thrombose,
en particulier comprenant en outre au moins un agent actif, en particulier choisi parmi les fibrinolytiques, de préférence la streptokinase, l'urokinase et/ou l'alteplase.

14. Système nanocarrier sensible aux stimuli, en particulier un liposome thermosensible, pour l'utilisation selon l'une des revendications précédentes en combinaison avec l'hyperthermie et/ou les ultrasons.

15. Un phosphatidyloligoglycérol stéréospécifique de la formule (IIa), 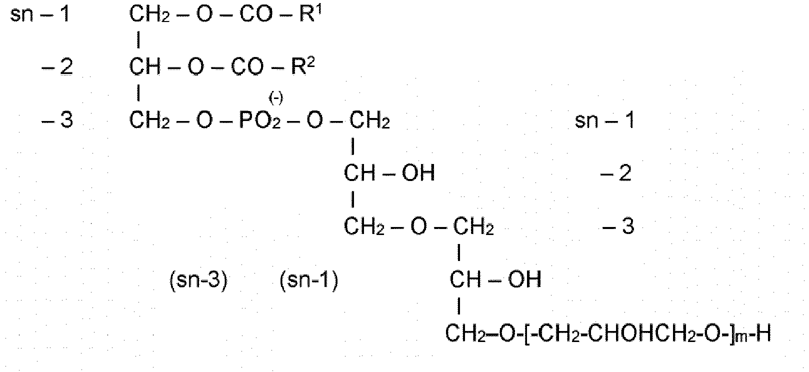 dans laquelle R¹ et R² représentent chacun indépendamment un radical hydrocarboné ayant de 12 à 24 atomes de carbone et m est un nombre entier de 0 à 50, dans laquelle la liaison du glycéride au groupe phosphate est stéréospécifique et est présente sous la forme d'une liaison sn-3 et la liaison du groupe phosphate à l'oligoglycérol est stéréospécifique et est présente sous la forme d'une liaison sn-1.
